# EUROPEAN PATENT APPLICATION

(11) **EP 4 643 846 A1**
(43) Date of publication of application: **05.11.2025**
(21) Application number: 23911774.0
(22) Date of filing: 15.12.2023
(51) Int. Cl.: A61K 8/893, A61K 8/35, A61K 8/37, A61K 8/49, A61K 8/89, A61K 8/894, A61Q 17/04

(54) **COSMETIC PRODUCT**

(30) Priority: 26.12.2022 JP 2022208172
(71) Applicant: SHIN-ETSU CHEMICAL CO., LTD., Tokyo 1000005 (JP)
(72) Inventor: MORIYA Hiroyuki, Annaka-shi, Gunma 379-0224 (JP); KIKUCHI Hiroko, Tokyo 100-0005 (JP)
(74) Representative: Mewburn Ellis LLP
(86) International application number: PCT/JP2023/045004
(87) International publication number: WO 2024/142993

(57) **Abstract**

Provided is a cosmetic product which contains an organopolysiloxane represented by formula (1), the cosmetic product having excellent long-term stability and make-up persistency even when an oil agent such as a silicone, a hydrocarbon oil and an ester, an organic ultraviolet absorber, and an oily component having a solid form at 25°C are blended.

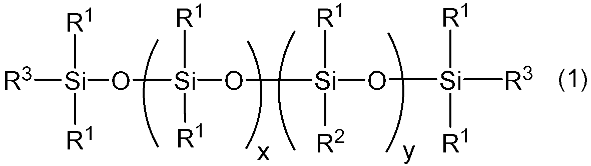

## Description

### TECHNICAL FIELD

The present invention relates to a cosmetic product containing an organopolysiloxane.

### BACKGROUND ART

Silicones, typified by dimethylpolysiloxanes, are frequently used as oils in cosmetic products in recent years to benefit from their characteristics, such as light and smooth feel, good spreadability, excellent water repellency, and high safety.

Among them, for example, cosmetic products contain volatile cyclic siloxanes, such as octamethylcyclotetrasiloxane (D4), decamethylcyclopentasiloxane (D5), and dodecamethylcyclohexasiloxane (D6), volatile linear siloxanes with a viscosity at 25°C of 0.65 to 2 mm²/s, and volatile siloxanes with branched siloxane chains, such as tristrimethylsiloxymethylsilane (M3T). Such cosmetic products excel in light and smooth feel, spreadability, and water repellency when applied to the skin (Patent Document 1). Some cosmetic ingredients, such as, in particular, ultraviolet absorbers and solid waxes, give a sticky or heavy feel after application. Volatile silicones are useful for relieving these feelings and providing a light and smooth feel.

However, these volatile silicones have poor compatibility with polar oils and cannot be mixed transparently. This problem may impair the feeling on use and the stability of cosmetic products. This tendency is particularly noticeable when an organic ultraviolet absorber is used. When, furthermore, the silicones have low affinity for oil ingredients, such as waxes, that are added to solidify cosmetic products, the oil ingredients are prevented from crystallization and fail to provide the desired hardness of preparations.

It is known to use, for example, phenyl-modified silicones and esters as compatibilizers in order to improve the above problem, that is, to increase the compatibility with polar oils, waxes, and the like. However, this approach results in a heavy feeling on use due to the influence of compatibilizers and the cosmetic products sometimes lose the characteristic light and smooth feel of volatile silicones. Furthermore, these compatibilizers have a higher refractive index than silicones and sometimes give an unnatural glare after application. Caprylyl methicone has a lighter feel and is sometimes used as a compatibilizer for the same purpose. However, caprylyl methicone does not provide sufficient compatibility in small dose and may impair the light feel of volatile silicones when used in a large amount.

To provide an alternative for formulating compositions containing volatile oils, Patent Document 2 proposes a composition that includes one or more acyclic volatile silicone oils and has specific evaporation characteristics. However, the specific description of formulations is limited to decamethyltetrasiloxane and dodecamethylpentasiloxane, and the disclosure does not consider the use of the acyclic silicone oils to achieve enhanced compatibility with polar oils, waxes, and the like.

Patent Document 3 discloses a study on the feeling on use of a composition in which an acyclic dimethylsiloxane is partially substituted by ethyl groups in place of methyl groups. However, this patent document does not consider whether the silicone, when added to cosmetic products, will provide enhanced compatibility with polar oils, waxes, and the like.

### PRIOR ART DOCUMENTS

### PATENT DOCUMENTS

Patent Document 1: WO 2001/015658
Patent Document 2: WO 2004/087077
Patent Document 3: US Patent Application Publication No. 2010/0144897

### SUMMARY OF INVENTION

### TECHNICAL PROBLEM

The present invention has been made in view of the circumstances discussed above. It is therefore an object of the present invention to provide a cosmetic product that exhibits excellent over-time stability and cosmetic durability even when the cosmetic product contains, for example, various oils, such as silicones, hydrocarbon oils, and esters, organic ultraviolet absorbers, and oil ingredients that are solid at 25°C.

### SOLUTION TO PROBLEM

The present inventors carried out intensive studies directed to achieving the above object and have found that the problems described above can be solved by adding to a cosmetic product a specific organopolysiloxane described below. Based on the finding, the present inventors have completed the present invention.

Thus, the present invention provides cosmetic products described below.
1. A cosmetic product containing an organopolysiloxane represented by formula (1) below: 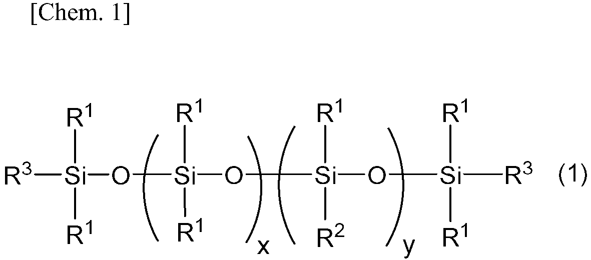 [wherein R¹ independently at each occurrence is a C₁-C₁₆ alkyl group; R² is an organic group represented by formula (2) below: 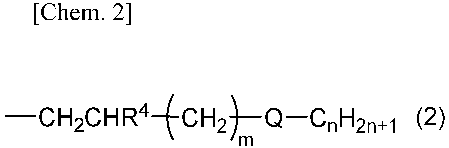 (wherein R⁴ is a hydrogen atom or a methyl group,
   Q is an organic group selected from linking groups represented by formulas (3) to (6) below: 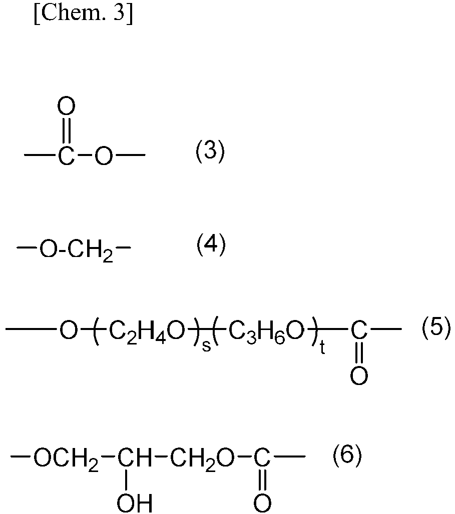 (wherein s is an integer of 0 to 3, t is an integer of 0 to 3, and the respective oxyalkylene groups are optionally bonded blockwise or randomly),
   when Q is formula (3), m is 8 and n is an integer of 1 to 22,
   when Q is formula (4), m is an integer of 1 to 10 and n is an integer of 7 to 22,
   when Q is formula (5), m is an integer of 1 to 10 and n is an integer of 7 to 22, and
   when Q is formula (6), m is an integer of 1 to 10 and n is an integer of 7 to 22);
   each R³ is R¹ or R²; x is an integer of 0 to 50; y is an integer of 0 to 50; at least one of R² or R³'s is an organic group represented by general formula (2); when y = 0, at least one of R³'s is R²; and the siloxane units with the subscripts x and y are optionally bonded blockwise or randomly].
2. The cosmetic product according to 1, wherein the organopolysiloxane has a surface tension of 28 mN/m or less and is nonvolatile.
3. The cosmetic product according to 1 or 2, further containing an organic ultraviolet absorber.
4. The cosmetic product according 1 to 3, wherein the organic ultraviolet absorber is one or more selected from ethylhexyl methoxycinnamate, diethylamino hydroxybenzoyl hexyl benzoate, octyl salicylate, polysilicone-15, t-butyl methoxydibenzoylmethane, oxybenzone, methylene bisbenzotriazolyl tetramethylbutylphenol, bisethylhexyloxyphenol methoxyphenyl triazine, and octocrylene.
5. The cosmetic product according to any one of 1 to 4, further containing an oil ingredient that is solid at 25°C.
6. The cosmetic product according to 5, wherein the oil ingredient that is solid at 25°C is one or more selected from polyethylene, ceresin, ozokerite, candelilla wax, carnauba wax, beeswax, microcrystalline wax, stearyl alcohol, behenyl alcohol, and cetanol.

### ADVANTAGEOUS EFFECTS OF INVENTION

The cosmetic product according to the present invention exhibits excellent over-time stability and cosmetic durability even when the cosmetic product contains, for example, various oils, such as silicones, hydrocarbon oils, and esters, organic ultraviolet absorbers, and oil ingredients that are solid at 25°C. Furthermore, the present invention can add various cosmetic effects.

### DESCRIPTION OF EMBODIMENTS

The present invention will be described in detail below without limiting the scope of the present invention to the following description. In the present invention, the name of an ingredient is written as the name cited in *Kesho̅hin Hyo̅ji Meisho̅* [Japanese Cosmetic Labeling Names] or in the International Nomenclature of Cosmetic Ingredients (INCI). When the names from *Kesho̅hin Hyo̅ji Meisho̅* and the INCI are the same, the INCI name or the name from *Kesho̅hin Hyo̅ji Meisho̅* is sometimes omitted.

### [Organopolysiloxanes]

An organopolysiloxane of the present invention is an organopolysiloxane represented by formula (1) below: 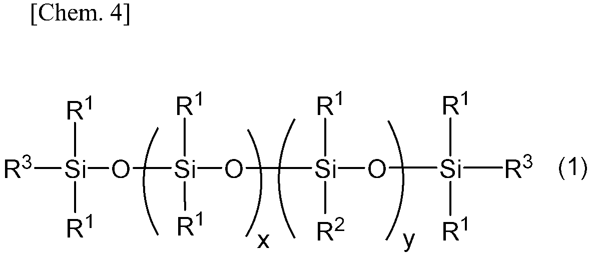 [wherein R¹ independently at each occurrence is a C₁-C₁₆ alkyl group; R² is an organic group represented by formula (2) below: 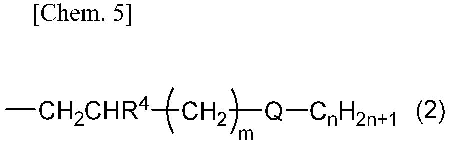
(wherein R⁴ is a hydrogen atom or a methyl group,
Q is an organic group selected from linking groups represented by formulas (3) to (6) below: 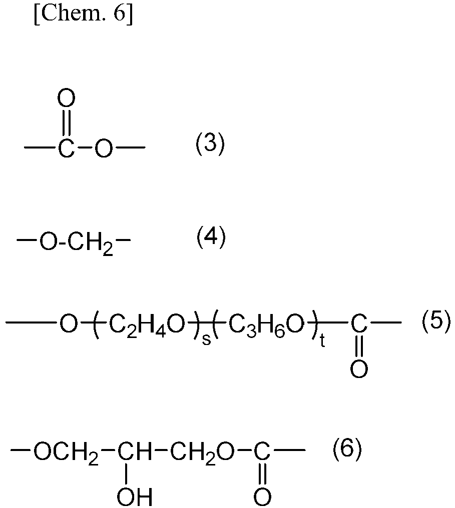
(wherein s is an integer of 0 to 3, t is an integer of 0 to 3, and the respective oxyalkylene groups are optionally bonded blockwise or randomly),
when Q is formula (3), m is 8 and n is an integer of 1 to 22,
when Q is formula (4), m is an integer of 1 to 10 and n is an integer of 7 to 22,
when Q is formula (5), m is an integer of 1 to 10 and n is an integer of 7 to 22, and
when Q is formula (6), m is an integer of 1 to 10 and n is an integer of 7 to 22);
each R³ is R¹ or R²; x is an integer of 0 to 50; y is an integer of 0 to 50; at least one of R² or R³'s is an organic group represented by general formula (2); when y = 0, at least one of R³'s is R²; and the siloxane units with the subscripts x and y are optionally bonded blockwise or randomly].

R¹ independently at each occurrence is a C₁-C₁₆ alkyl group, preferably a C₁-C₁₂ alkyl group. Examples of the alkyl groups include linear alkyl groups, such as methyl group, ethyl group, propyl group, butyl group, pentyl group, hexyl group, heptyl group, octyl group, nonyl group, decyl group, undecyl group, and dodecyl group, and cyclic alkyl groups, such as cyclopentyl group and cyclohexyl group. Preferred examples of R¹ include methyl group, ethyl group, hexyl group, and dodecyl group, with methyl group being particularly preferable.

R² is represented by formula (2) below: 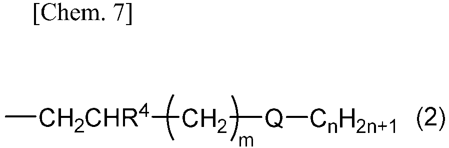
(wherein R⁴ is a hydrogen atom or a methyl group, and
Q is an organic group selected from linking groups represented by formulas (3) to (6) below: 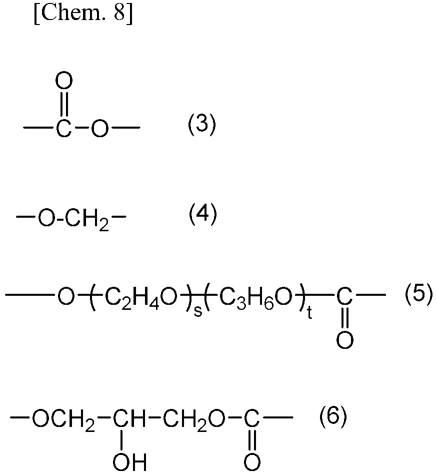
(wherein s is an integer of 0 to 3, t is an integer of 0 to 3, and the respective oxyalkylene groups are optionally bonded blockwise or randomly).

R⁴ is a hydrogen atom or a methyl group, and is preferably a hydrogen atom.

When Q is formula (3), m is 8 and n is an integer of 1 to 22, preferably 1 to 18.

When Q is formula (4), m is an integer of 1 to 10, preferably an integer of 1 to 8, and n is an integer of 7 to 22, preferably an integer of 8 to 18.

When Q is formula (5), m is an integer of 1 to 10, preferably 1 to 6, and n is an integer of 7 to 22, preferably 8 to 18. The subscript s is an integer of 0 to 3, preferably 0 to 2, and t is an integer of 0 to 3, preferably 0 to 2. The sum of s + t is preferably 1 or greater.
The respective oxyalkylene groups are optionally bonded blockwise or randomly. Specific examples of the linking groups include those having the following structures:

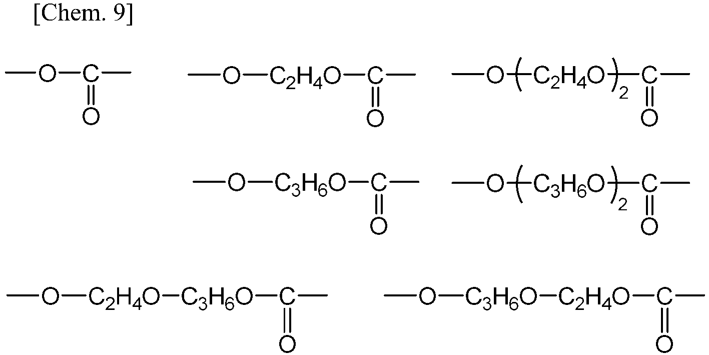

When Q is formula (6), m is an integer of 1 to 10, preferably 1 to 8, and n is an integer of 7 to 22, preferably 8 to 18.

The linking group Q is preferably represented by formula (3), (4), or (5), and is more preferably represented by formula (3) or (4).

R³'s are each R¹ or R², and examples thereof are the same as those of R¹ and R² described above. R³ is preferably a methyl group or a group represented by formula (2), and is more preferably a group represented by formula (2). When y = 0, at least one of R³'s is R². At least one of R² and R³'s is an organic group represented by formula (2), and the number of the organic groups represented by formula (2) in the molecule is preferably 1 to 40, more preferably 1 to 30.

The subscript x is an integer of 0 to 50, preferably 0 to 30, and y is an integer of 0 to 50, preferably 0 to 30. The siloxane units with the subscripts x and y are optionally bonded blockwise or randomly, and the order in which the siloxane units are bonded is not limited to the above. The subscripts x and y may be each 1 or greater. Even when x = y = 0, the compound is treated as an organopolysiloxane in the present invention.

### [Production methods]

For example, the organopolysiloxane represented by formula (1) may be produced by a hydrosilylation reaction between the corresponding organohydrogenpolysiloxane and a long-chain alkyl-containing compound having a terminal carbon-carbon double bond. The groups R¹ in the resultant compound may include a small amount of hydrogen atoms from the unreacted hydrosilyl groups that remain after the reaction, or a small amount of hydroxyl groups formed by the reaction. Specifically, the amount of such hydrogen atoms or hydroxyl groups may be 10% or less, preferably 5% or less, of the number of all the groups R¹.

Preferably, the organopolysiloxane of the present invention has a surface tension of 28 mN/m or less and is nonvolatile. The surface tension is more preferably 15 to 28 mN/m. The surface tension is a value measured by the Wilhelmy method at 25°C using a surface tensiometer. In the present invention, "nonvolatile" means that a 0.5 g sample has a weight loss of 10 wt% or less after being allowed to stand in a 60 mm diameter aluminum petri dish at 25°C for 3 hours under atmospheric pressure (1,013 hPa).

### [Cosmetic products]

A cosmetic product of the present invention contains the organopolysiloxane described above. The organopolysiloxanes may be used singly, or two or more may be used in combination. The content thereof is not particularly limited but is preferably 1 to 70 wt% of the cosmetic product. By virtue of the incorporation of the organopolysiloxane, the cosmetic product attains excellent over-time stability and cosmetic durability even when the cosmetic product contains, for example, various oils, such as silicones, hydrocarbon oils, and esters, organic ultraviolet absorbers, and oil ingredients that are solid at 25°C. Furthermore, the organopolysiloxane does not destroy the smooth feel peculiar to silicones, and the cosmetic product exhibits good spreadability, is free from rough feeling, and can form a film that is smooth and light. Furthermore, the cosmetic product has a light and smooth feel, spreads well, excels in water repellency, and can achieve a good feeling on use.

### [Organic ultraviolet absorbers]

The organopolysiloxane of the present invention has excellent compatibility with organic ultraviolet absorbers. Thus, the cosmetic product, when containing an organic ultraviolet absorber, attains a good feeling on use and exhibits excellent over-time stability and cosmetic durability.

Any organic ultraviolet absorbers that are usually added to cosmetic products may be used without limitation. Such organic ultraviolet absorbers may be used singly, or two or more may be used in combination. Specific examples include oxybenzone-1 (INCI: Benzophenone-1), oxybenzone-2 (INCI: Benzophenone-2), oxybenzone-3 (INCI: Benzophenone-3), oxybenzone-4 (INCI: Benzophenone-4), oxybenzone-5 (INCI: Benzophenone-5), oxybenzone-6 (INCI: Benzophenone-6), oxybenzone-9 (INCI: Benzophenone-9), Homosalate (INCI), octocrylene, t-butyl methoxydibenzoylmethane, ethylhexyl salicylate, diethylamino hydroxybenzoyl hexyl benzoate, polysilicone-15, octyl dimethoxybenzylidenedioxoimidazolidinepropionate (INCI: Ethylhexyl Dimethoxybenzylidene Dioxoimidazolidine Propionate), Terephthalylidene Dicamphor Sulfonic Acid (INCI), Ethylhexyltriazone (INCI), methylbis(trimethylsiloxy)silylisopentyl trimethoxycinnamate (INCI: Isopentyl Trimethoxycinnamate Trisiloxane), Drometrizole Trisiloxane (INCI), Ethylhexyl Dimethyl PABA (INCI), Isopropyl Methoxycinnamate (INCI), ethylhexyl methoxycinnamate, bisethylhexyloxyphenol methoxyphenyl triazine, Phenylbenzimidazole Sulfonic Acid (INCI), methylene bisbenzotriazolyl tetramethylbutylphenol, Glyceryl Ethylhexanoate Dimethoxycinnamate (INCI), Glyceryl PABA (INCI), Diisopropyl Methyl Cinnamate (INCI), Cinoxate (INCI), and Ethylhexyl Dimethoxybenzylidene Dioxoimidazolidine Propionate (INCI). Furthermore, a UVA absorber (such as, for example, diethylamino hydroxybenzoyl hexyl benzoate) and a UVB absorber (such as, for example, ethylhexyl methoxycinnamate) may be used in combination, or absorbers belonging to the same type may be combined appropriately.

In particular, for the reason that high ultraviolet absorbing effects and excellent compatibility with the organopolysiloxanes of the present invention can be obtained, the organic ultraviolet absorber is preferably one or more selected from Ethylhexyl Methoxycinnamate (INCI), Diethylamino Hydroxybenzoyl Hexyl Benzoate (INCI), Ethylhexyl Salicylate (INCI), Polysilicone-15 (INCI), t-butyl methoxydibenzoylmethane (INCI: Butyl Methoxydibenzoylmethane), oxybenzone (INCI: Benzophenone), Methylene Bisbenzotriazolyl Tetramethylbutylphenol (INCI), Bisethylhexyloxyphenol Methoxyphenyl Triazine (INCI), and Octocrylene (INCI).

When the organic ultraviolet absorber is added, the content thereof in the cosmetic product is preferably 1 to 40 wt%, and more preferably 2 to 10 wt%.

### [Volatile silicones]

Volatile silicones are silicone oils that are at least partially volatile at 25°C. Any volatile silicones that are usually added to cosmetic products may be used without limitation. Volatile silicones may be used singly, or two or more may be used in combination. Specific examples include decamethyltetrasiloxane, undecamethylpentasiloxane, decamethylcyclopentasiloxane, dodecamethylcyclohexasiloxane, diethyloctamethyltetrasiloxane, and tristrimethylsiloxymethylsilane.

### [Oil ingredients that are solid at 25°C]

The organopolysiloxane of the present invention has high affinity with oil ingredients. Even when used in combination with an oil ingredient that is solid at 25°C, the organopolysiloxane does not inhibit the solidification of the oil ingredient and the cosmetic product that is obtained can be easily prepared as, for example, a stick-shaped solid preparation.

The oil ingredient that is solid at 25°C is not particularly limited, and such oil ingredients may be used singly, or two or more may be used in combination. Specific examples include waxes, hydrocarbons, esters, higher alcohols, and higher fatty acids that are each solid at 25°C.

The oil ingredients that are solid at 25°C preferably have a melting point of 40°C or above, more preferably 60 to 110°C. Any such ingredients that are usually added to cosmetic products may be used without limitation, with examples including waxes, hydrocarbons, esters, higher alcohols, and higher fatty acids.

Specific examples include vegetable waxes, such as carnauba wax, sugarcane wax, candelilla wax, refined candelilla wax, rice wax, wood wax, jojoba wax, kapok wax, rice bran wax, bayberry fruit wax, shea butter, cacao butter, Rhus Succedanea Fruit Wax (INCI), Montan Wax (INCI), and hydrogenated castor oil isostearate; animal waxes, such as beeswax, beef tallow, beef bone fat, Lard (INCI), Horse Fat (INCI), mutton tallow, Lanolin (INCI), Ericerus pela wax, shellac wax, and spermaceti; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffins, polyethylene, ceresin, ozokerite, and microcrystalline wax; higher alcohols, such as stearyl alcohol, behenyl alcohol, and cetanol; wax esters, such as synthetic beeswax; amino acid stearyl alcohols, such as dioctyldodecyl lauroyl glutamate, dioctyldodecyl lauroyl glutamate, and dioctyldodecyl lauroyl glutamate; fatty acids, such as stearic acid and behenic acid; silicone waxes, such as acrylic silicone resins of acrylic-silicone graft or block copolymers (for example, acrylic-silicone graft copolymers: KP-561P and KP-562P, manufactured by Shin-Etsu Chemical Co., Ltd.); and derivatives thereof. One, or two or more selected from the above are preferably used.

In particular, it is preferable to use one or more selected from Polyethylene (INCI), Ceresin (INCI), Ozokerite (INCI), candelilla wax (INCI: Euphorbia Cerifera (Candelilla) Wax), carnauba wax (INCI: Copernicia Cerifera (Carnauba) Wax), Beeswax (INCI), Microcrystalline Wax (INCI), Stearyl Alcohol (INCI), Behenyl Alcohol (INCI), and cetanol (INCI: Cetyl Alcohol).

When the oil ingredient that is solid at 25°C is added, the content thereof in the cosmetic product is preferably 0.5 to 35 wt%, and more preferably 5 to 15 wt%.

The cosmetic product of the present invention may contain various optional ingredients that are usually added to cosmetic products.

### <Other optional ingredients>

The cosmetic product may contain other optional ingredients, for example, (1) oils, (2) aqueous ingredients, (3) surfactants, (4) powders, (5) compositions including a crosslinked organopolysiloxane and an oil that is liquid at room temperature, (6) film-forming agents, (7) antiperspirants, (8) antimicrobial agents, and (9) other additives. These may be used singly, or two or more may be used in appropriate combination. When any of the ingredients described hereinabove also appears as an optional ingredient, the description given hereinabove takes precedence.

### (1) Oils

The oils may be semi-solid or liquid oils other than the oil ingredients that are solid at 25°C. Examples include natural animal and vegetable oils and fats, semi-synthetic oils and fats, hydrocarbon oils, higher fatty acids, higher alcohols, esters, silicone oils other than the organopolysiloxanes that are the essential ingredients for achieving the advantageous effects of the present invention, and fluorinated oils.

### • Natural animal and vegetable oils and fats, and semi-synthetic oils and fats

Examples of the natural animal and vegetable oils and the semi-synthetic oils include natural vegetable oils, such as avocado oil (INCI: Persea Gratissima (Avocado) Oil), linseed oil (INCI: Linum Usitatissimum (Linseed) Seed Oil), almond oil (INCI: Prunus Amygdalus Dulcis (Sweet Almond) Oil), perilla oil, olive oil (INCI: Olea Europaea (Olive) Fruit Oil), Torreya californica oil (INCI: Torreya Californica (California Nutmeg) Oil), citronella grass oil (INCI: Cymbopogon Nardus (Citronella) Oil), Torreya Nucifera Seed Oil (INCI), apricot kernel oil (INCI: Kyounin Yu), wheat germ oil (INCI: Triticum Vulgare (Wheat) Germ Oil), sesame oil (INCI: Sesamum Indicum (Sesame) Seed Oil), rice germ oil (INCI: Oryza Sativa (Rice) Germ Oil), rice bran oil (INCI: Oryza Sativa (Rice) Bran Oil), sasanqua oil (INCI: Camellia Kissi Seed Oil), safflower oil (INCI: Carthamus Tinctorius (Safflower) Seed Oil), soybean oil (INCI: Glycine Soja (Soybean) Oil), tea seed oil (INCI: Camellia Sinensis Seed Oil), camellia oil (INCI: Camellia Japonica Seed Oil), evening primrose oil (INCI: Oenothera Biennis (Evening Primrose) Oil), rapeseed oil (INCI: RAPE SHUSHI YU), germ oils, for example, corn germ oil (INCI: Zea Mays (Corn) Germ Oil), persic oil, palm oil (INCI: Elaeis Guineensis (Palm) Oil), palm kernel oil (INCI: Elaeis Guineensis (Palm) Kernel Oil), castor oil (INCI: Ricinus Communis (Castor) Seed Oil), sunflower oil (INCI: Helianthus Annuus (Sunflower) Seed Oil), grape seed oil (INCI: Vitis Vinifera (Grape) Seed Oil), Jojoba seed oil (INCI: Simmondsia Chinensis (Jojoba) Seed Oil), macadamia seed oil (INCI: Macadamia Ternifolia Seed Oil), meadowfoam oil (INCI: Limnanthes Alba (Meadowfoam) Seed Oil), cotton seed oil (INCI: Gossypium Herbaceum (Cotton) Seed Oil), coconut oil (INCI: Cocos Nucifera (Coconut) Oil), and peanut oil (INCI: Arachis Hypogaea (Peanut) Oil); natural animal oils, such as Shark Liver Oil (INCI), Cod Liver Oil (INCI), Fish Liver Oil (INCI), Turtle Oil (INCI), Mink Oil (INCI), and egg yolk oil (INCI: Egg Oil); and semi-synthetic oils and fats, such as Hydrogenated Coconut Oil (INCI) and liquid lanolin (INCI: Lanolin Oil).

### • Hydrocarbon oils

Examples of the hydrocarbon oils include linear or branched hydrocarbon oils. The hydrocarbon oils may be volatile or nonvolatile.

Specific examples include alkanes, such as Olefin Oligomers (INCI), isoparaffins, for example, (C13, 14) Isoparaffins (INCI), Isododecane (INCI), Undecane (INCI), Dodecane (INCI), Isohexadecane (INCI), Hydrogenated Polyisobutene (INCI), Squalane (INCI), Mineral Oil (INCI), Coconut Alkanes (INCI), (C13-15) Alkanes (INCI), and vaseline (INCI: Petrolatum).

### • Higher fatty acids

Examples of the higher fatty acids include Oleic Acid (INCI), Linoleic Acid (INCI), Linolenic Acid (INCI), Arachidonic Acid (INCI), Eicosapentaenoic Acid (INCI), Docosahexaenoic Acid (INCI), Isostearic Acid (INCI), and Hydroxystearic Acid (INCI).

### • Higher alcohols

For example, the higher alcohols are preferably C₆ and higher alcohols. Specific examples of the higher alcohols include Lauryl Alcohol (INCI), Myristyl Alcohol (INCI), Palmityl Alcohol (INCI), Stearyl Alcohol (INCI), Behenyl Alcohol (INCI), Oleyl Alcohol (INCI), Isostearyl Alcohol (INCI), Octyldodecanol (INCI), Cholesterol (INCI), Phytosterols (INCI), and Batyl Alcohol (INCI).

### • Ester oils

Examples of the ester oils include Diisobutyl Adipate (INCI), dihexyldecyl adipate, Diheptylundecyl Adipate (INCI), n-alkyl glycol monoisostearates, such as Isostearyl Isostearate (INCI), Isocetyl Isostearate (INCI), Trimethylolpropane Triisostearate (INCI), Glycol Diethylhexanoate (INCI), Cetyl Ethylhexanoate (INCI), Trimethylolpropane Triethylhexanoate (INCI), Pentaerythrityl Tetraethylhexanoate (INCI), cetyl octanoate (INCI: Cetyl Ethylhexanoate), octyldodecyl esters, such as octyldodecyl stearoyloxystearate (INCI: Octyldodecyl Stearoyl Stearate), Oleyl Oleate (INCI), Octyldodecyl Oleate (INCI), Decyl Oleate (INCI), neopentyl glycol dioctanoate (INCI: Neopentyl Glycol Diethylhexanoate), Neopentyl Glycol Dicaprate (INCI), Diisostearyl Malate (INCI), Triethyl Citrate (INCI), Diethylhexyl Succinate (INCI), Amyl Acetate (INCI), Ethyl Acetate (INCI), Butyl Acetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), palmitic acid esters, such as Isopropyl Palmitate (INCI), ethylhexyl palmitate (INCI: Ethylhexyl Isopalmitate), and hexyldecyl palmitate (INCI: Isocetyl Palmitate, Hexyldecyl Palmitate), Cholesteryl Hydroxystearate (INCI), myristic acid esters, such as Isopropyl Myristate (INCI), Octyldodecyl Myristate (INCI), and Myristyl Myristate (INCI), Ethylhexyl Laurate (INCI), Hexyl Laurate (INCI), Dioctyldodecyl Lauroyl Glutamate (INCI), and Isopropyl Lauroyl Sarcosinate (INCI).

Among the ester oils, glyceride oils include Triethylhexanoin (INCI), glyceryl tri(caprylate/caprate) (INCI: Caprylic/Capric Triglyceride), Cocoglyceride (INCI), triglyceryl (caprylate/caprate/succinate) (INCI: Caprylic/Capric/Succinic Triglyceride), and Caprylic/Capric Glycerides (INCI).

### • Silicone oils

The silicone oils may be silicone oils other than the organopolysiloxanes that are the essential ingredients for achieving the advantageous effects of the present invention.

Examples include alkyl-modified silicones, such as Dimethicone (INCI), Trisiloxane (INCI), Methyltrimethicone (INCI), Ethyltrisiloxane (INCI), Ethylmethicone (INCI), and Hexyldimethicone (INCI), long-chain alkyl-modified silicones, such as Caprylyl Methicone (INCI), low-viscosity to high-viscosity, linear or branched organopolysiloxanes, such as Phenyl Trimethicone (INCI), Diphenyl Dimethicone (INCI), Diphenylsiloxyphenyl Trimethicone (INCI), Tetraphenyldimethyldisiloxane (INCI), and methylhydrogenpolysiloxane, cyclic organopolysiloxanes, such as Cyclotetrasiloxane (INCI), Cyclopentasiloxane (INCI), and Cyclohexasiloxane (INCI), amino-modified organopolysiloxanes, such as Amodimethicone (INCI) and Aminopropyl Dimethicone (INCI), pyrrolidone-modified organopolysiloxanes, such as PCA Dimethicone (INCI), pyrrolidonecarboxylic acid-modified organopolysiloxanes, silicone rubbers, such as high degree of polymerization gum-like dimethylpolysiloxanes, gum-like amino-modified organopolysiloxanes, and gum-like dimethylsiloxane-methylphenylsiloxane copolymers, low-viscosity organopolysiloxane solutions of a silicone gum or rubber, amino acid-modified silicones, fluorine-modified silicones, silicone resins, and silicone resin solutions.

Examples of the commercial silicone oils include KF-96L-1cs, KF-96L-1.5cs, KF-96L-2cs, KF-96A-6cs, KF-4422, KF-4418, KF-54, KF-54HV, KF-56A, and KF-995 manufactured by Shin-Etsu Chemical Co., Ltd.

### • Fluorinated oils

Examples of the fluorinated oils include Perfluorodecalin (INCI), Perfluorononyl Dimethicone (INCI), and Perfluoromethylcyclopentane (INCI).

### (2) Aqueous ingredients

Any aqueous ingredients that are usually added to cosmetic products may be used without limitation. Specific examples of the aqueous ingredients include water, moisturizers, and water-soluble polymer compounds. These may be used singly, or two or more may be used in appropriate combination.

Examples of water include purified water commonly used in cosmetic products, distilled water from fruits and plants, and seawater, hot spring water, and peat water defined in *Kesho̅hin Hyo̅ji Meisho̅.*

Examples of the moisturizers include lower alcohols, preferably those having 2 to 5 carbon atoms, such as ethanol (INCI: Alcohol) and isopropanol (INCI: Isopropyl Alcohol); sugar alcohols, such as Sorbitol (INCI), Maltose (INCI), and Xylitol (INCI); polyhydric alcohols, such as BG (INCI: Butylene Glycol), PG (INCI: Propylene Glycol), DPG (INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; Glucose (INCI), Glyceryl Glucoside (INCI), Betaine (INCI), Sodium Chondroitin Sulfate (INCI), Sodium PCA (INCI), Methyl Gluceth-10 (INCI), and Methyl Gluceth-20 (INCI). Examples further include sterols, such as Cholesterol (INCI), sitosterol (INCI: Beta-Sitosterol), Phytosterols (INCI), and Lanosterol (INCI). Examples of the moisturizers further include hyaluronic acid, egg yolk lecithin, soybean lecithin, phosphatidylcholine, phosphatidylethanolamine, phosphatidylserine, phosphatidylglycerol, phosphatidylinositol, and sphingophospholipid. The amount in which the moisturizer is added is preferably 1 to 90 wt%, more preferably 3 to 50 wt%, and still more preferably 5 to 20 wt% of the whole of the cosmetic product.

Examples of the water-soluble polymer compounds include natural water-soluble polymer compounds, such as carrageenan, hyaluronates, and xanthan gum; semi-synthetic water-soluble polymer compounds, such as hydroxyethyl cellulose, hydroxypropylmethyl cellulose, and carboxymethyl cellulose; synthetic water-soluble polymer compounds, such as polyvinyl alcohol, polyvinylpyrrolidone, and carboxyvinyl polymer; and inorganic water-soluble polymer compounds, such as bentonite and laponite. The amount in which the water-soluble polymer compound is added is preferably 0.01 to 1 wt% of the whole of the cosmetic product.

### (3) Surfactants

The surfactants may be nonionic, anionic, cationic, or amphoteric and any surfactants usually added to cosmetic products may be used without limitation. In particular, preferred surfactants are partially crosslinked polyether-modified silicones, partially crosslinked polyglycerin-modified silicones, linear or branched polyoxyethylene-modified organopolysiloxanes, linear or branched polyoxyethylene polyoxypropylene-modified organopolysiloxanes, linear or branched polyoxyethylene/alkyl co-modified organopolysiloxanes, linear or branched polyoxyethylene polyoxypropylene/alkyl co-modified organopolysiloxanes, linear or branched polyglycerin-modified organopolysiloxanes, and linear or branched polyglycerin/alkyl co-modified organopolysiloxanes.

In these surfactants, the content of hydrophilic polyoxyethylene groups, polyoxyethylene polyoxypropylene groups, or polyglycerin residues is preferably 10 to 70 wt% of the molecule.

Examples of the partially crosslinked polyether-modified silicones and the partially crosslinked polyglycerin-modified silicones include (Dimethicone/(PEG-10/15) Crosspolymer (INCI), (PEG-15/Lauryl Dimethicone) Crosspolymer (INCI) , (PEG-10/Lauryl Dimethicone) Crosspolymer (INCI), (PEG-15/Lauryl Polydimethylsiloxyethyl Dimethicone) Crosspolymer (INCI), (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

When the partially crosslinked polyether-modified silicone or the partially crosslinked polyglycerin-modified silicone is used, the crosslinked organopolysiloxane preferably forms a composition with an oil that is liquid at room temperature in such a manner that the crosslinked organopolysiloxane is swollen with its own weight or more of the liquid oil.

The liquid oil may be the organopolysiloxane in the present invention or may be the optional ingredient (1), specifically, a liquid silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Examples of the commercial crosslinked organopolysiloxanes swollen with liquid oils include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z manufactured by Shin-Etsu Chemical Co., Ltd.

Exemplary surfactants that are not crosslinked organopolysiloxanes include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), and Lauryl Polyglyceryl-3 Polydimethylsiloxyethyl Dimethicone (INCI).

Exemplary commercial products include KF-6011, KF-6011P, KF-6012, KF-6015, KF-6017, KF-6043, KF-6028, KF-6038, KF-6048, KF-6100, KF-6104, KF-6106, KF-6105, and KF-6115 manufactured by Shin-Etsu Chemical Co., Ltd.

For any types of surfactants, the amount is preferably 0.1 to 20 wt% of the whole of the cosmetic product. When the amount is 0.1 wt% or more, the surfactant can fully fulfill its dispersing and emulsifying functions. When the amount is 20 wt% or less, the cosmetic product will not give a sticky feeling on use and is therefore preferable. The HLB of the surfactant is not particularly limited but is preferably 2 to 14.5 in order to maintain the water resistance of the cosmetic product.

### (4) Powders

Examples of the powders include coloring pigments, inorganic powders, metal powders, organic powders, and inorganic/organic composite powders. The powders will be specifically described below.

### • Coloring pigments

The coloring pigments are not particularly limited and any pigments that are usually added to give colors to cosmetic products may be used. Examples include red iron oxide (INCI: Iron Oxides), yellow iron oxide (INCI: Iron Oxides), white titanium oxide (INCI: Titanium Dioxide), black iron oxide (INCI: Iron Oxides), Ultramarines (INCI), iron blue (INCI: Ferric Ferrocyanide, Ferric Ammonium Ferrocyanide), Manganese Violet (INCI), Cobalt Titanium Oxide (INCI), chromium hydroxide (INCI: Chromium Hydroxide Green), chromium oxide (INCI: Chromium Oxide Greens), Cobalt Aluminum Oxide (INCI), fired (titanium/titanium oxide) (INCI: Titanium/Titanium Dioxide), Lithium Cobalt Titanate (INCI), sintered (iron oxide/titanium oxide), composites doped with dissimilar metals, such as iron oxide doped titanium oxide (INCI: Iron Oxides, Titanium Dioxide), Titanium Nitride (INCI), ferrous hydroxide (INCI: Iron Hydroxide), inorganic brown pigments, such as γ-iron oxide, inorganic yellow pigments, such as loess, and colored pigments, such as lakes of tar dyes and lakes of natural dyes.

The pigments may have any shapes, such as spherical, approximately spherical, rod-like, spindle-like, petal-like, strip-like, and amorphous shapes. The geometric forms of the pigments are not particularly limited as long as the pigments can impart colors to the cosmetic products.

### • Inorganic powders

Examples of the inorganic powders include fine particles of zirconium oxide (INCI: Zirconium Dioxide), Zinc Oxide (INCI), Cerium Oxide (INCI), Magnesium Oxide (INCI), Barium Sulfate (INCI), Calcium Sulfate (INCI), Magnesium Sulfate (INCI), Calcium Carbonate (INCI), Magnesium Carbonate (INCI), Talc (INCI), Mica (INCI), Kaolin (INCI), Synthetic Fluorphlogopite (INCI), synthetic ferrous phlogopite, black mica (INCI: Biotite), Potassium Silicate (INCI), Silica (INCI), Aluminum Silicate (INCI), Magnesium Silicate (INCI), Magnesium Aluminum Silicate (INCI), Calcium Silicate (INCI), Aluminum Calcium Sodium Silicate (INCI), Lithium Magnesium Sodium Silicate (INCI), Sodium Magnesium Silicate (INCI), Calcium Aluminum Borosilicate (INCI), Calcium Sodium Borosilicate (INCI), Hydroxyapatite (INCI), Bentonite (INCI), Montmorillonite (INCI), Hectorite (INCI), Zeolite (INCI), Alumina (INCI), Aluminum Hydroxide (INCI), Boron Nitride (INCI), and Glass (INCI).

Inorganic coloring pearl pigments include pearl agents, such as Mica (INCI) coated with titanium oxide (INCI: Titanium Dioxide) and Synthetic Fluorophlogopite (INCI) coated with titanium oxide (INCI: Titanium Dioxide), and pearl pigments, such as Bismuth Oxychloride (INCI), Bismuth Oxychloride (INCI) coated with titanium oxide (INCI: Titanium Dioxide), Talc (INCI) coated with titanium oxide (INCI: Titanium Dioxide), fish scale flakes, and coloring mica coated with titanium oxide (INCI: Titanium Dioxide). These pigments may be surface-treated by a known method generally used for cosmetic products or may be free from such surface treatment.

### • Metal powders

Examples of the metal powders include metal fine particles of aluminum (INCI: Aluminum, Aluminum Powder), copper (INCI: Copper Powder), silver (INCI: Silver Powder), and Gold (INCI).

### • Organic powders

Examples of the organic powders include powders of silicone, polyamide, polyacrylic acid/acrylic acid ester, polyester, Polyethylene (INCI), Polypropylene (INCI), Polystyrene (INCI), styrene/acrylic acid copolymer, divinylbenzene/styrene copolymer, polyurethane, vinyl resin, urea resin, melamine resin, benzoguanamine, polymethylbenzoguanamine, tetrafluoroethylene, polymethyl methacrylate, Cellulose (INCI), Silk (INCI), nylon, phenolic resin, epoxy resin, and polycarbonate.

In particular, examples of the silicones include silicone resin particles; Polymethylsilsesquioxane (INCI), silicone rubber powder, silicone resin-coated silicone rubber powder; (Vinyl Dimethicone/Methicone Silsesquioxane) Crosspolymer (INCI), (Diphenyl Dimethicone/Vinyldiphenyl Dimethicone/Silsesquioxane) Crosspolymer (INCI), Polysilicone-1 Crosspolymer (INCI), and Polysilicone-22 (INCI).

Examples of the commercial silicone powders include KMP-590, KMP-591, KMP-592, KMP-597, KMP-598, KSP-100, KSP-101, KSP-102, KSP-105, KSP-300, KSP-411, KSP-441, KM-9729, and KM-440 manufactured by Shin-Etsu Chemical Co., Ltd.

Examples further include metal soaps. Specific examples include powders of Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), and Potassium Cetyl Phosphate (INCI).

Examples further include organic dyes. Specific examples include tar dyes, such as red 3, red 104 (1) (INCI: Red 28, Red 28 Lake), red 106, red 201 (INCI: Red 6), red 202 (INCI: Red 7), red 204, red 205, red 220 (INCI: Red 34), red 226 (INCI: Red 30), red 227 (INCI: Red 33, RED 33 Lake), red 228 (INCI: Red 36), red 230 (1) (INCI: Red 22, Red 22 Lake), red 230 (2), red 401, red 505, yellow 4 (INCI: Yellow 5), yellow 5 (INCI: Yellow 6, Yellow 6 Lake), yellow 202 (1) (INCI: Yellow 8), yellow 203 (INCI: Yellow 10, Yellow 10 Lake), yellow 204 (INCI: Yellow 11), yellow 401, blue 1 (INCI: Blue 1, Blue 1 Lake), blue 2, blue 201, blue 205 (INCI: Blue 4), blue 404, green 3 (INCI: Green 3, Green 3 Lake), green 201 (INCI: Green 5), green 202 (INCI: Green 6), green 204 (INCI: Green 8), green 205, orange 201 (INCI: Orange 5), orange 203 (INCI: Pigment Orange 5), orange 204, orange 205 (INCI: Orange 4, Orange 4 Lake), orange 206 (INCI: Orange 10), and orange 207 (INCI: Orange 11), and natural dyes, such as Cochineal (INCI), Laccaic Acid (INCI), safflower red (INCI: Carthamus Tinctorius (Safflower) Flower Extract), Lithospermum Officinale Root Extract (INCI), gardenia yellow, and gardenia blue (INCI: Hydrolyzed Gardenia Florida Extract).

### • Inorganic/organic composite powders

Examples of the inorganic/organic composite powders include composite powders in which the surface of an inorganic powder is coated with an organic powder by a publicly known method.

The powders described hereinabove may be surface-treated. From the point of view of the water resistance of the cosmetic product, the surface treatment agent is preferably one that can impart hydrophobicity. The hydrophobicity-imparting agent is not particularly limited, and examples thereof include silicone treatment agents, waxes, paraffins, organic fluorine compounds, such as perfluoroalkyls and phosphate salts, surfactants, amino acids, such as N-acylglutamic acid, and metal soaps, such as aluminum stearate and magnesium myristate. Silicone treatment agents are more preferable. Examples thereof include silanes or silylating agents, such as Triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and Acrylates/Tridecyl Acrylate/Triethoxysilylpropyl Methacrylate/Dimethicone Methacrylate Copolymer (INCI). Specific examples of the silicone treatment agents include AES-3083, KF-99P, KF-9901, KF-9908, KF-9909, KP-574, and KP-541 manufactured by Shin-Etsu Chemical Co., Ltd.

The surface hydrophobizing agents may be used singly, or two or more may be used in combination. Specific examples of the surface-treated coloring pigments include KTP-09 series, in particular, KTP-09W, 09R, 09Y, and 09B manufactured by Shin-Etsu Chemical Co., Ltd.

### (5) Compositions including a crosslinked organopolysiloxane and an oil that is liquid at room temperature

In the composition including a crosslinked organopolysiloxane and an oil that is liquid at room temperature, the crosslinked organopolysiloxane is preferably swollen by containing its own weight or more of the liquid oil. The liquid oil may be the organopolysiloxane in the present invention or may be the optional ingredient (1), specifically, a liquid silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Unlike the ingredients (3), the ingredients (5) are compounds that do not have a polyether or polyglycerin structure in the molecular structure. Specific examples include (Dimethicone/Vinyl Dimethicone) Crosspolymer (INCI), (Dimethicone/Phenyl Vinyl Dimethicone) Crosspolymer (INCI), (Vinyl Dimethicone/Lauryl Dimethicone) Crosspolymer (INCI), and (Lauryl Polydimethylsiloxyethyl Dimethicone/Bisvinyl Dimethicone) Crosspolymer (INCI).

Examples of the commercial compositions including a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

### (6) Film-forming agents

The film-forming agent is added mainly for the purpose of further increasing the durability of the effects of the cosmetic product. The film-forming agents are not particularly limited but are preferably silicone compositions from the point of view of imparting water repellency. Specifically, for example, trimethylsiloxysilicate, acrylic-silicone coating agents, silicone-modified norbornenes, and silicone-modified pullulans may be used.

Examples of the silicone compositions as the film-forming agents include Trimethylsiloxysilicate (INCI), (Acrylates/Dimethicone) Copolymer (INCI), (Norbornene/Tris(trimethylsiloxy)silylnorbornene) Copolymer (INCI), and pullulan tri(trimethylsiloxy)silylpropylcarbamate (INCI: Trimethylsiloxysilylcarbamoyl Pullulan).

The film-forming agent may be added to the cosmetic product after being previously dissolved into an oil that is liquid at room temperature. The liquid oil may be the organopolysiloxane in the present invention or may be the optional ingredient (1), specifically, a liquid silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), Squalane (INCI), and Butyl Acetate (INCI).

Specific examples of the commercial silicone film-forming agents include KF-7312J, KP-545, KP-549, KP-543, NBN-30-ID, TSPL-30-ID, and TSPL-30-D5 manufactured by Shin-Etsu Chemical Co., Ltd.

### (7) Antiperspirants

When the cosmetic product according to the present invention is a deodorant, an antiperspirant may be optionally added. The antiperspirant is not particularly limited as long as it shrinks the skin pores to suppress perspiration. General such ingredients may be widely used. Examples include halogenated hydroxyaluminums, such as chlorohydroxyaluminum and allantoin chlorohydroxyaluminum, aluminum halides, such as aluminum chloride, allantoin aluminum salt, tannic acid, persimmon tannin, aluminum potassium sulfate, zinc oxide, zinc p-phenolsulfonate, burnt alum, tetrachloro(Al/zirconium) hydrate, and trichlorohydrex glycine (Al/zirconium). In order to obtain high effects, in particular, the antiperspirant active ingredient is preferably selected from the group consisting of aluminum halides, halogenated hydroxyaluminums, and complexes or mixtures thereof with zirconyl oxyhalides and zirconyl hydroxyhalides.

The antiperspirant may be used as a solution in water, or a powder thereof may be added directly to the preparation. Commercially available antiperspirants may be used. The commercially available product may be in the form of a mixed ingredient containing other ingredients. The content of the antiperspirant is not particularly limited and may be changed appropriately depending on the amounts of other ingredients that are blended. In order to obtain a deodorant with excellent antiperspirant effects and to reduce the skin irritation of the deodorant, the content is preferably in the range of 0.001 to 30 wt%, and more preferably in the range of 0.01 to 20 wt% of the whole of the deodorant.

### (8) Antimicrobial agents

The antimicrobial agents are not particularly limited as long as the ingredients can provide deodorizing effects by suppressing the proliferation of bacteria resident on the skin that produce odor-causing substances. For example, such antimicrobial agents as triclosan, benzalkonium chloride, benzethonium chloride, chlorhexidine hydrochloride, chlorhexidine gluconate, halocarban, and isomethylphenol are generally used. Furthermore, for example, crude drug-derived essential oils or extracts having antibacterial properties, such as green tea dry distillation extract, may be added. Examples of the antimicrobial agents that are crude drug-derived essential oils or extracts having deodorizing effects include green tea extract, lavender extract, Scutellaria baicalensis Georgi extract, Coptis japonica extract, Phellodendron Amurense Bark extract, Artemisia capillaris extract, Aloe arborescens extract, Sophora flavescens root extract, Sasa veitchii leaf extract, Allium sativum extract, Hamamelis extract, black tea extract, Salvia officinalis leaf extract, Zanthoxylum fruit extract, Zingiber Officinale root extract, Acorus Calamus root extract, Hedera Helix extract, Houttuynia Cordata extract, Prunus Persica fruit extract, Prunus Persica leaf extract, Mentha Piperita leaf extract, Cnidium Officinale root extract, Eucalyptus Globulus leaf extract, Arachis Hypogaea seedcoat extract, Ganoderma Lucidum extract, and Sanguisorba Officinalis root extract. The plant extracts may be used singly, or two or more may be used in combination.

### (9) Other additives

Other additives include oil-soluble gelling agents, ultraviolet absorbing and scattering agents, preservatives, fragrances, salts, antioxidants, pH adjusters, chelating agents, algefacients, anti-inflammatory agents, skin beautifying ingredients (such as whitening agents, cell activators, skin roughness improvers, circulation promoting ingredients, skin astringents, and antiseborrheic agents), vitamins, amino acids, nucleic acids, hormones, and clathrate compounds.

### • Oil-soluble gelling agents

Examples of the oil-soluble gelling agents include metal soaps, such as aluminum stearate, magnesium stearate, and zinc myristate; amino acid derivatives, such as Lauroyl Glutamic Acid (INCI) and α,γ-di-n-butylamine; dextrin fatty acid esters, such as Dextrin Palmitate (INCI), Dextrin Isostearate (INCI), Dextrin Myristate (INCI), inulin stearate (INCI: Stearoyl Inulin), and Dextrin Palmitate/Ethylhexanoate (INCI); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organically modified clay minerals of hectorite, such as Disteardimonium Hectorite (INCI) and Stearalkonium Hectorite (INCI); and Stearalkonium Bentonite (INCI).

### • Ultraviolet absorbing and scattering agents

Examples of the ultraviolet absorbing and scattering agents include particles that absorb and scatter ultraviolet rays, such as titanium oxide fine particles, iron-containing titanium oxide fine particles, zinc oxide fine particles, cerium oxide fine particles, and composites thereof. These ultraviolet absorbing and scattering particles may be dispersed in an oil beforehand.

The oil may be the organopolysiloxane in the present invention or may be the optional ingredient (1), specifically, a liquid silicone oil, a hydrocarbon oil, an ester oil, a natural animal or vegetable oil, a semi-synthetic oil, or a fluorinated oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Specific examples of the oil dispersions of the ultraviolet absorbing and scattering particles include SPD series (product name), in particular, SPD-T5, T5L, Z5, Z5L, T6, Z6, T7, and Z7L manufactured by Shin-Etsu Chemical Co., Ltd.

### • Preservatives

Examples of the preservatives include p-oxybenzoic acid alkyl esters, benzoic acid, sodium benzoate, sorbic acid, potassium sorbate, and phenoxyethanol. Examples of the antimicrobial agents include benzoic acid, salicylic acid, carbolic acid, sorbic acid, p-oxybenzoic acid alkyl esters, p-chloro-m-cresol, hexachlorophene, trichlorocarbanilide, photosensitizers, and phenoxyethanol.

### • Fragrances

The fragrances include natural fragrances and synthetic fragrances. Examples of the natural fragrances include plant fragrances separated from, for example, flowers, leaves, wood, and pericarps; and animal fragrances, such as musk and civet. Examples of the synthetic fragrances include hydrocarbons, such as monoterpenes; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

The salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Examples of the organic acid salts include salts of organic acids, such as acetic acid, dehydroacetic acid, citric acid, malic acid, succinic acid, ascorbic acid, and stearic acid. Examples of the amine salts and the amino acid salts include salts of amines, such as triethanolamine, and salts of amino acids, such as glutamic acid. Furthermore, use may be made of, for example, salts of hyaluronic acid and chondroitin sulfuric acid, aluminum zirconium glycine complex, and acid-alkali neutral salts used in cosmetic formulations.

### • Antioxidants

The antioxidants are not particularly limited. Examples include carotenoids, ascorbic acid and salts thereof, ascorbyl stearate, tocophenol, tocophenol acetate, tocopherol, p-t-butylphenol, butylhydroxyanisole, dibutylhydroxytoluene, phytic acid, ferulic acid, thiotaurine, hypotaurine, sulfite salts, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • pH adjusters

Examples of the pH adjusters include lactic acid, citric acid, glycolic acid, succinic acid, tartaric acid, dl-malic acid, potassium carbonate, sodium hydrogen carbonate, and ammonium hydrogen carbonate.

### • Chelating agents

Examples of the chelating agents include alanine, sodium edetate, sodium polyphosphate, sodium metaphosphate, and phosphoric acid.

### • Algefacients

Examples of the algefacients include L-menthol and camphor.

### • Anti-inflammatory agents

Examples of the anti-inflammatory agents include allantoin, glycyrrhizic acid and salts thereof, glycyrrhetinic acid and stearyl glycyrrhetinate, tranexamic acid, and azulene.

### • Skin beautifying ingredients

Examples of the skin beautifying ingredients include whitening agents, such as placenta extract, arbutin, glutathione, and Saxifraga Sarmentosa extract; cell activators, such as royal jelly, photosensitizers, cholesterol derivatives, and calf blood extract; skin roughness improvers; circulation promoting ingredients, such as nonylic acid vanillylamide, benzyl nicotinate, β-butoxyethyl nicotinate, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Examples of the vitamins include vitamins A, such as vitamin A oils, retinol, retinyl acetate, and retinyl palmitate; vitamins B, including vitamins B₂, such as riboflavin, riboflavin butyrate, and flavin adenine nucleotide, vitamins B₆, such as pyridoxine hydrochloride, pyridoxine dioctanoate, and pyridoxine tripalmitate, vitamin B₁₂ and derivatives thereof, and vitamin B₁₅ and derivatives thereof; vitamins C, such as L-ascorbic acid, L-ascorbyl dipalmitate, sodium L-ascorbyl-2-sulfate, and dipotassium L-ascorbyl diphosphate; vitamins D, such as ergocalciferol and cholecalciferol; vitamins E, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinamide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Examples of the nucleic acids include deoxyribonucleic acid.

### • Hormones

Examples of the hormones include estradiol and ethenyl estradiol.

### • Clathrate compounds

Examples of the clathrate compounds include cyclodextrin.

The cosmetic product of the present invention may be applied to various manufactured articles, such as, but not particularly limited to, lotions, beauty essences, milky lotions, creams, hair care products, foundations, makeup bases, sunscreens, concealers, blush colors, lipsticks, glosses, balms, mascaras, eye shadows, eyeliners, body makeups, deodorants, and nail cosmetic products. Among these, in particular, makeup cosmetic products and preparations with sunscreen effects are preferable, with examples including foundations, makeup bases, sunscreens, concealers, blush colors, lipsticks, and glosses. The type of formulation of the cosmetic product of the present invention may be selected from various types of formulations, such as liquids, creams, solids, pastes, gels, mousses, souffles, clays, powders, and sticks.

The cosmetic products described above may be in the form of emulsion, oil-based, or water-based. When an oily feel is desired, an oil-based composition or an emulsion is selected. The emulsion may be in any form of O/W emulsion, W/O emulsion, O/W/O emulsion, or W/O/W emulsion. When a fresh feel is desired, a water-based composition or a powder composition may be selected. In any of these cases, good-quality cosmetic products may be obtained.

### EXAMPLES

Hereinafter, the present invention will be described in detail by presenting Production Examples, and Examples and Comparative Examples of the cosmetic products of the present invention. However, the present invention is not limited to the following Examples. Unless otherwise specified, "%" in the compositions below means "wt%" and indicates the wt% of an ingredient relative to the weight of the total of each example taken as 100 wt%. The amount indicates the amount of the product described. The orders of bonding of siloxanes are not limited to those described below. The surface tension is a value measured by the Wilhelmy method at 25°C using a surface tensiometer (device name: DY-700, manufactured by Kyowa Interface Science Co., Ltd.). In the formulas below, n-Bu denotes an n-butyl group. In the formulas, terminals that are not bonded and are not specifically described are "-CH₃", and the terminal of a double bond is "CH₂=CH₂-".

### [Production Example 1]

A reactor was charged with 144 g of the compound represented by the following formula: 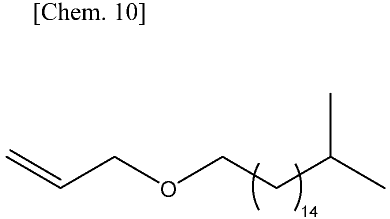 and 0.05 g of an ethanol solution (0.5%) of chloroplatinic acid divinyldisiloxane complex. The mixture was heated to 80°C while performing stirring. There was added dropwise 100 g of 1,1,1,3,5,5,5-heptamethyltrisiloxane while maintaining the temperature at 100°C or below. The mixture was aged at 100°C for 5 hours. Disappearance of hydrosilyl groups was confirmed by ¹H-NMR. The reaction liquid was stripped under reduced pressure at 140°C to remove unreacted materials and the like. A pale yellow, transparent organopolysiloxane represented by the formula below was thus obtained. The surface tension of the organopolysiloxane obtained was 25.6 mN/m. There was no weight change at 25°C in air, and the compound was nonvolatile.

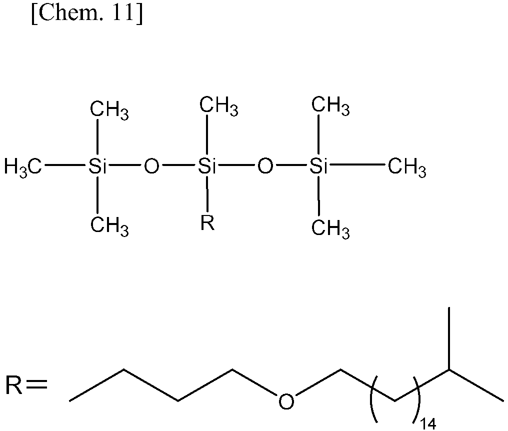

### [Production Example 2]

A reactor was charged with 431 g of the compound represented by the following formula: 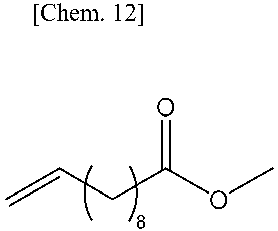 and 0.08 g of an ethanol solution (0.5%) of chloroplatinic acid divinyldisiloxane complex. The mixture was heated to 80°C while performing stirring. There was added dropwise 350 g of 1,1,1,3,5,5,5-heptamethyltrisiloxane while maintaining the temperature at 100°C or below. The mixture was aged at 100°C for 5 hours. Disappearance of hydrosilyl groups was confirmed by ¹H-NMR. The reaction liquid was stripped under reduced pressure at 140°C to remove unreacted materials and the like. A pale yellow, transparent organopolysiloxane represented by the formula below was thus obtained. The surface tension of the organopolysiloxane obtained was 24.2 mN/m. There was no weight change at 25°C in air, and the compound was nonvolatile.

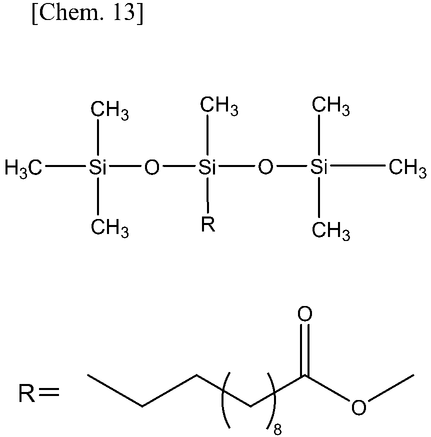

### [Production Example 3]

A reactor was charged with 520 g of the compound represented by the following formula: 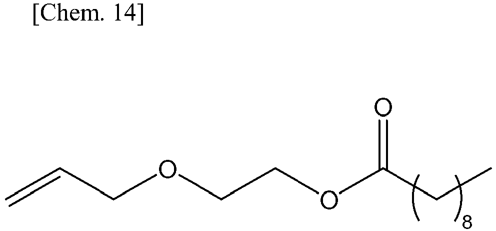 and 0.1 g of an ethanol solution (0.5%) of chloroplatinic acid divinyldisiloxane complex. The mixture was heated to 80°C while performing stirring. There was added dropwise 160 g of tetramethyldisiloxane while maintaining the temperature at 100°C or below. The mixture was aged at 100°C for 5 hours. Disappearance of hydrosilyl groups was confirmed by ¹H-NMR. The reaction liquid was stripped under reduced pressure at 140°C to remove unreacted materials and the like. A pale yellow, transparent organopolysiloxane represented by the formula below was thus obtained. The surface tension of the organopolysiloxane obtained was 25.5 mN/m. There was no weight change at 25°C in air, and the compound was nonvolatile.

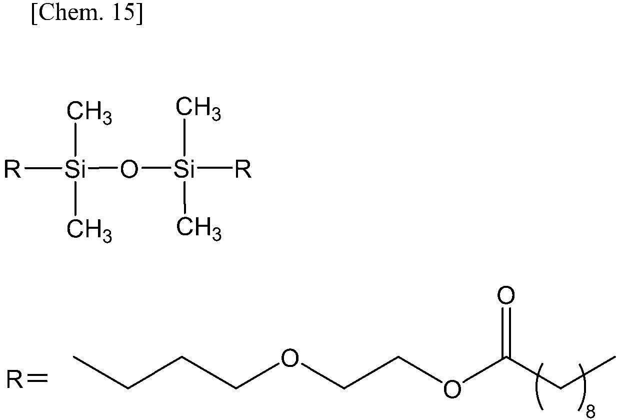

### [Production Example 4]

A reactor was charged with 1200 g of the compound represented by the following formula: 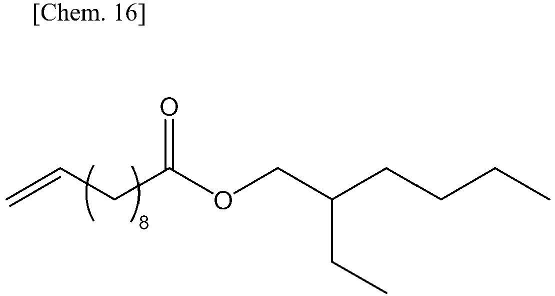 and 0.1 g of an ethanol solution (0.5%) of chloroplatinic acid divinyldisiloxane complex. The mixture was heated to 80°C while performing stirring. There was added dropwise 550 g of methylhydrogensiloxane represented by the following formula while maintaining the temperature at 100°C or below. 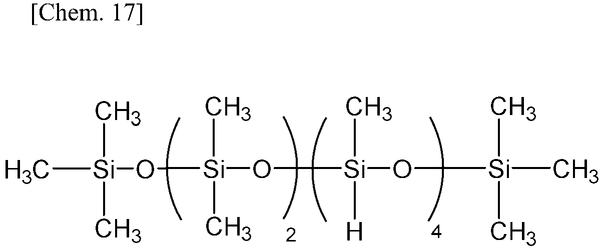 The mixture was aged at 100°C for 5 hours. Disappearance of hydrosilyl groups was confirmed by ¹H-NMR. The reaction liquid was stripped under reduced pressure at 140°C to remove unreacted materials and the like. A pale yellow, transparent organopolysiloxane represented by the formula below was thus obtained. The surface tension of the organopolysiloxane obtained was 27.0 mN/m. There was no weight change at 25°C in air, and the compound was nonvolatile.

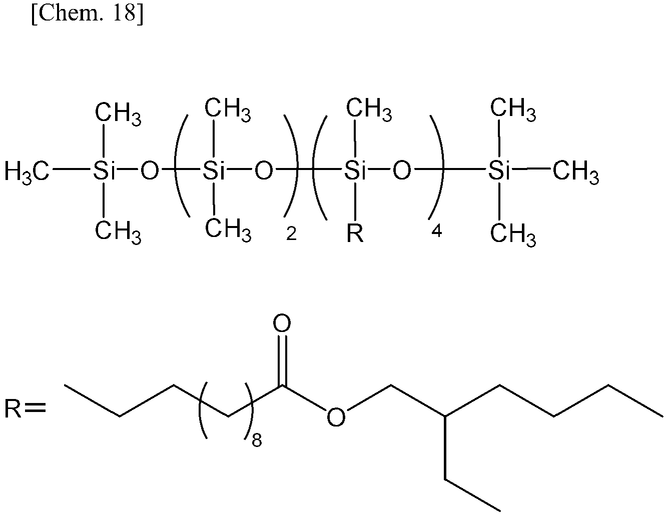

### [Production Example 5]

A reactor was charged with 690 g of the compound represented by the following formula: 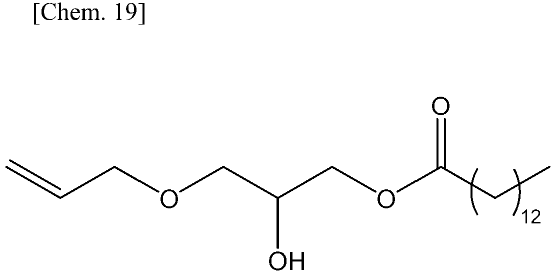 and 0.2 g of an ethanol solution (0.5%) of chloroplatinic acid divinyldisiloxane complex. The mixture was heated to 80°C while performing stirring. There was added dropwise 280 g of methylhydrogensiloxane represented by the following formula while maintaining the temperature at 100°C or below. 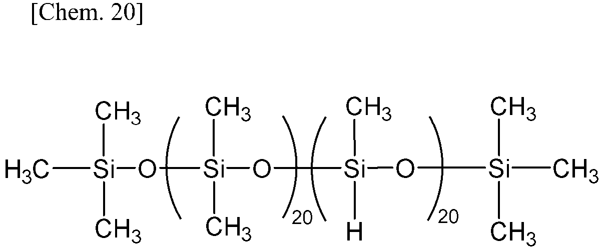 The mixture was aged at 100°C for 5 hours. Disappearance of hydrosilyl groups was confirmed by ¹H-NMR. The reaction liquid was stripped under reduced pressure at 140°C to remove unreacted materials and the like. A pale yellow, transparent organopolysiloxane represented by the formula below was thus obtained. The surface tension of the organopolysiloxane obtained was 27.9 mN/m. There was no weight change at 25°C in air, and the compound was nonvolatile.

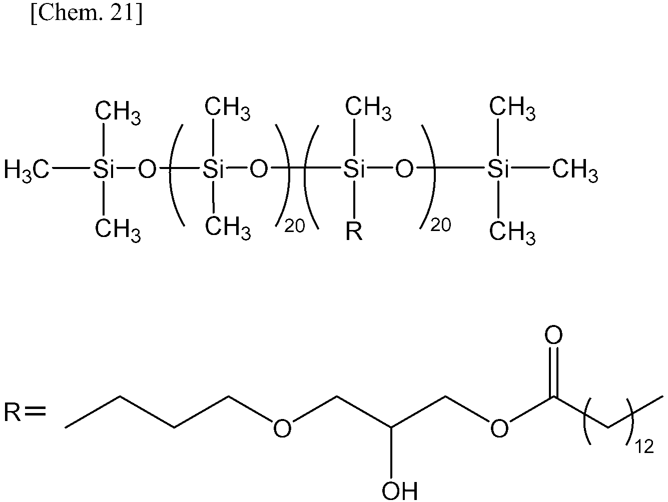

### [Production Example 6]

A reactor was charged with 220 g of the compound represented by the following formula: 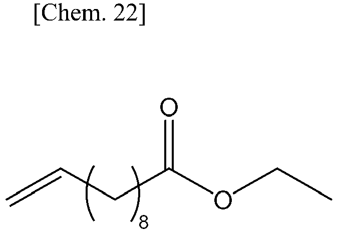 and 0.08 g of an ethanol solution (0.5%) of chloroplatinic acid divinyldisiloxane complex. The mixture was heated to 80°C while performing stirring. There was added dropwise 220 g of 1,1,1,3,5,5,5-heptamethyltrisiloxane while maintaining the temperature at 100°C or below. The mixture was aged at 100°C for 5 hours. Disappearance of hydrosilyl groups was confirmed by ¹H-NMR. The reaction liquid was stripped under reduced pressure at 140°C to remove unreacted materials and the like. A pale yellow, transparent organopolysiloxane of the following structure was thus obtained. The surface tension of the organopolysiloxane obtained was 24.3 mN/m. There was no weight change at 25°C in air, and the compound was nonvolatile.

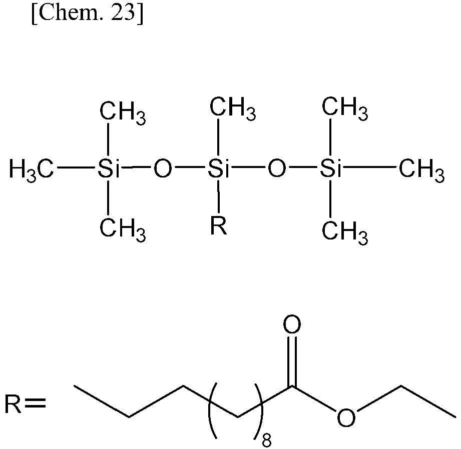

### [Production Example 7]

A reactor was charged with 440 g of the compound represented by the following formula: 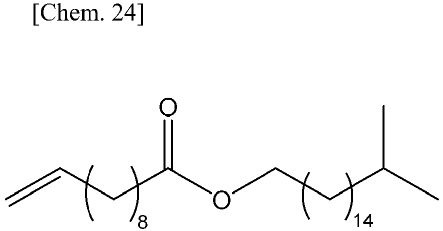 and 0.1 g of an ethanol solution (0.5%) of chloroplatinic acid divinyldisiloxane complex. The mixture was heated to 80°C while performing stirring. There was added dropwise 410 g of methylhydrogensiloxane represented by the following formula while maintaining the temperature at 100°C or below. 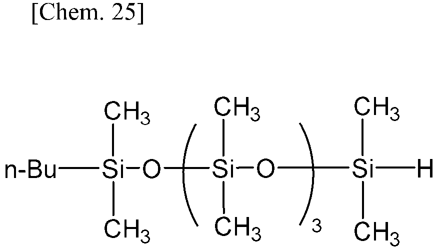 The mixture was aged at 100°C for 5 hours. Disappearance of hydrosilyl groups was confirmed by ¹H-NMR. The reaction liquid was stripped under reduced pressure at 140°C to remove unreacted materials and the like. A pale yellow, transparent organopolysiloxane represented by the formula below was thus obtained. The surface tension of the organopolysiloxane obtained was 26.5 mN/m. There was no weight change at 25°C in air, and the compound was nonvolatile. 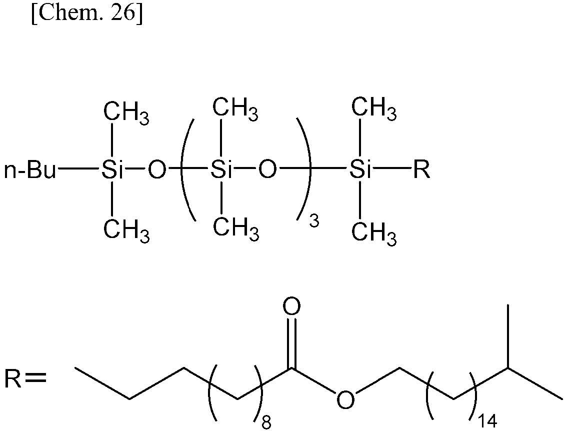 n-Bu is an n-butyl group.

The organopolysiloxanes obtained in Production Examples 1 and 2 were evaluated as follows.

### [Solubility tests between volatile silicone and ethylhexyl methoxycinnamate (organic ultraviolet absorber) with organopolysiloxanes]

Solubility tests were performed between a volatile silicone and ethylhexyl methoxycinnamate (an organic ultraviolet absorber) with the organopolysiloxanes obtained in Production Examples 1 and 2. The results are described in Table 1.

### (Evaluation method)

The organopolysiloxanes obtained in Production Examples 1 and 2, a volatile silicone, specifically, undecamethylpentasiloxane (KF-96L-2cs, manufactured by Shin-Etsu Chemical Co., Ltd.), and ethylhexyl methoxycinnamate (also written as OMC) were collected in the weight ratios described in Table 1 and were mixed by shaking at room temperature. The state of dissolution was observed the next day. The state of dissolution is rated as below.
○: Dissolved and uniformly dispersed
×: Separated

**[Table 1]**

| Weight ratios | Test 1 | Test 2 | Test 3 | Test 4 | Test 5 | Test 6 | Test 7 |
|---|---|---|---|---|---|---|---|
| Undecamethylpentasiloxane (KF-96L-2cs) (Note 1) | 14 | 12 | 13 | 12 | 13 | 12 | 12 |
| Ethylhexyl methoxycinnamate | 7 | 7 | 7 | 7 | 7 | 7 | 7 |
| Organopolysiloxane of Production Example 1 | | 2 | | | | | |
| Organopolysiloxane of Production Example 2 | | | 1 | 2 | | | |
| Diphenylsiloxyphenyl trimethicone KF-56A (Note 2) | | | | | 1 | 2 | |
| Caprylyl methicone | | | | | | | 2 |
| State of dissolution | × | ○ | ○ | ○ | × | ○ | × |

| | | | | | | | |
|---|---|---|---|---|---|---|---|
| (Note 1) KF-96L-2cs manufactured by Shin-Etsu Chemical Co., Ltd. (Note 2) KF-56A manufactured by Shin-Etsu Chemical Co., Ltd. | | | | | | | |

As described in Table 1, the organopolysiloxanes obtained in Production Examples 1 and 2 of the present invention were shown to have comparable or higher compatibilizing properties than the conventionally used diphenylsiloxyphenyl trimethicone (a compatibilizer) and to have higher compatibilizing properties than caprylyl methicone in uniformly mixing a volatile silicone and ethylhexyl methoxycinnamate.

### [Solubility tests between organopolysiloxanes and oil ingredients solid at 25°C]

The solubility between the organopolysiloxanes obtained in Production Examples 1 and 2, and oil ingredients that are solid at 25°C was evaluated by the method described below. The evaluation results are described in Table 2.

### (Evaluation method)

An oil ingredient that is solid at 25°C and the organopolysiloxane obtained in Production Example 1 or 2, or other silicone were collected in a weight ratio of 1:9, and the mixture was heated at 90°C for 20 minutes. The state of dissolution was visually observed and was evaluated according to the following criteria. The results are described in Table 2.
⊚: Uniformly dissolved
△: Uniformly dispersed (turbid)
×: Separated

**[Table 2]**

| | Ceresin | Candelilla wax | Carnauba wax |
|---|---|---|---|
| Organopolysiloxane of Production Example 1 | ⊚ | ⊚ | ⊚ |
| Organopolysiloxane of Production Example 2 | ⊚ | ⊚ | ⊚ |
| Diphenylsiloxyphenyl trimethicone KF-56A | ⊚ | ⊚ | ⊚ |
| Caprylyl methicone | ⊚ | △ | × |

As described in Table 2, the organopolysiloxanes obtained in Production Examples 1 and 2 of the present invention were shown to offer comparable or higher solubility than the conventionally used compatibilizers when mixed uniformly with an oil ingredient that is solid at 25°C.

The advantageous effects of the present invention will be described below based on Examples and Comparative Examples of the cosmetic products.

### [Examples 1 and 2, and Comparative Examples 1 to 3] W/O sunscreens

W/O sunscreens were prepared in accordance with the production method described below using the organopolysiloxanes from Production Examples 1 and 2 in Examples 1 and 2, and without using these organopolysiloxanes in Comparative Examples 1 to 3. The compositions of Examples 1 and 2, and Comparative Examples 1 to 3 are described in Table 3.

**[Table 3]**

| Composition (%) | | Example 1 | Example 2 | Comp. Example 1 | Comp. Example 2 | Comp. Example 3 |
|---|---|---|---|---|---|---|
| 1 | Organopolysiloxane of Production Example 1 | 5.0 | - | - | - | - |
| 2 | Organopolysiloxane of Production Example 2 | - | 4.0 | - | - | - |
| 3 | KF-56A (Note 1) | - | - | 5.0 | - | - |
| 4 | Caprylyl methicone | - | - | - | 5.0 | 11.0 |
| 5 | Dodecamethylpentasiloxane | 9.0 | 10.0 | 9.0 | 9.0 | 3.0 |
| 6 | Ethylhexyl methoxycinnamate | 7.5 | 7.5 | 7.5 | 7.5 | 7.5 |
| 7 | Diethylamino hydroxybenzoyl hexyl benzoate | 2.0 | 2.0 | 2.0 | 2.0 | 2.0 |
| 8 | Octocrylene | 2.5 | 2.5 | 2.5 | 2.5 | 2.5 |
| 9 | Ethylhexyl salicylate | 1.0 | 1.0 | 1.0 | 1.0 | 1.0 |
| 10 | KSG-210 (Note 2) | 3.5 | 3.5 | 3.5 | 3.5 | 3.5 |
| 11 | KSG-18A (Note 3) | 3.0 | 3.0 | 3.0 | 3.0 | 3.0 |
| 12 | KF-6048 (Note 4) | 1.5 | 1.5 | 1.5 | 1.5 | 1.5 |
| 13 | 1,3-Butylene glycol | 3.0 | 3.0 | 3.0 | 3.0 | 3.0 |
| 14 | Sodium citrate | 0.5 | 0.5 | 0.5 | 0.5 | 0.5 |
| 15 | Sodium chloride | 0.5 | 0.5 | 0.5 | 0.5 | 0.5 |
| 16 | Ethanol | 5.0 | 5.0 | 5.0 | 5.0 | 5.0 |
| 17 | Purified water | 56.0 | 56.0 | 56.0 | 56.0 | 56.0 |
| Total | | 100.0 | 100.0 | 100.0 | 100.0 | 100.0 |

| | | | | | | |
|---|---|---|---|---|---|---|
| (Note 1) Shin-Etsu Chemical Co., Ltd.: Diphenylsiloxyphenyl trimethicone (Note 2) Shin-Etsu Chemical Co., Ltd.: Mixture of 70-80% dimethicone + 20-30% (dimethicone/(PEG-10/15)) crosspolymer (Note 3) Shin-Etsu Chemical Co., Ltd.: Mixture of 80-85% diphenylsiloxyphenyl trimethicone + 15-20% crosslinked phenyl-modified dimethylpolysiloxane (Note 4) Shin-Etsu Chemical Co., Ltd.: Cetyl PEG/PPG-10/1 dimethicone | | | | | | |

### [Production method]

Ingredients 1 to 12 were mixed together uniformly by stirring. Separately, ingredients 13 to 16 were uniformly dissolved into ingredient 17, and the mixture was gently added to the mixture of ingredients 1 to 12 described above. The resultant mixture was stirred to form an emulsion, which was then loaded into a predetermined container. A W/O sunscreen was thus obtained.

The W/O sunscreens obtained were used by a panel of ten experts to test (1) lightness of feel, (2) spreadability, (3) smoothness of film, and (4) absence of glare according to the ratings below. The scores were averaged and the results were evaluated based on the criteria below. The results are described in Table 4.

Furthermore, the cosmetic products were observed (5) after being allowed to stand at 40°C for one month and (6) after being allowed to stand at 5°C for one month. The results are described in Table 4.

### (Ratings)

| | |
|---|---|
| 5 Points: | Very good |
| 4 Points: | Good |
| 3 Points: | Fair |
| 2 Points: | Rather poor |
| 1 Point: | Poor |

The average score was evaluated based on the following ○ × criteria.

### (Evaluation criteria)

⊚: The average score is 4.5 points or more.
○: The average score is 3.5 points or more and less than 4.5 points.
△: The average score is 2.5 points or more and less than 3.5 points.
×: The average score is 1.5 points or more and less than 2.5 points.
××: The average score is less than 1.5 points.

**[Table 4]**

| No. | Evaluation items | Example 1 | Example 2 | Comp. Example 1 | Comp. Example 2 | Comp. Example 3 |
|---|---|---|---|---|---|---|
| (1) | Lightness of feel | ○ | ⊚ | × | ⊚ | × |
| (2) | Spreadability | ⊚ | ⊚ | ⊚ | ⊚ | ⊚ |
| (3) | Smoothness of film | ⊚ | ⊚ | △ | ⊚ | ○ |
| (4) | Absence of glare | ○ | ○ | × | ○ | ○ |
| (5) | State of cosmetic product (40°C) | Stable | Stable | Stable | Separated | Stable |
| (6) | State of cosmetic product (5°C) | Stable | Stable | Stable | Separated | Stable |

As clear from Table 4, the sunscreens of Examples 1 and 2 as compared to Comparative Examples 1 and 3 were demonstrated to have a light feel and good spreadability, and to form a smooth and glare-free cosmetic film. Furthermore, these sunscreens were shown to have excellent storage stability. The sunscreen of Comparative Example 2 had a good feel but was poor in storage stability.

### [Examples 3 and 4, and Comparative Examples 4 and 5] Stick-type W/O foundations

Stick-type W/O foundations were prepared in accordance with the production method described below using the organopolysiloxanes from Production Examples 1 and 2 in Examples 3 and 4, and without using these organopolysiloxanes in Comparative Examples 4 and 5. The compositions of Examples 3 and 4, and Comparative Examples 4 and 5 are described in Table 5.

**[Table 5]**

| Composition (%) | | Example 3 | Example 4 | Comp. Example 4 | Comp. Example 5 |
|---|---|---|---|---|---|
| 1 | Organopolysiloxane of Production Example 1 | 8.0 | - | - | - |
| 2 | Organopolysiloxane of Production Example 2 | - | 8.0 | - | - |
| 3 | KF-56A (Note 1) | - | - | 8.0 | - |
| 4 | Caprylyl methicone | - | - | - | 8.0 |
| 5 | Dimethylpolysiloxane (6 cs) | 11.5 | 11.5 | 11.5 | 11.5 |
| 6 | Ethylhexyl methoxycinnamate | 7.0 | 7.0 | 7.0 | 7.0 |
| 7 | Ceresin | 5.5 | 5.5 | 5.5 | 5.5 |
| 8 | Candelilla wax | 2.0 | 2.0 | 2.0 | 2.0 |
| 9 | KSG-710 (Note 2) | 4.0 | 4.0 | 4.0 | 4.0 |
| 10 | KF-6038 (Note 3) | 1.5 | 1.5 | 1.5 | 1.5 |
| 11 | KTP-09W (Note 4) | 8.5 | 8.5 | 8.5 | 8.5 |
| 12 | KTP-09R (Note 4) | 1.6 | 1.6 | 1.6 | 1.6 |
| 13 | KTP-09Y (Note 4) | 0.7 | 0.7 | 0.7 | 0.7 |
| 14 | KTP-09B (Note 4) | 0.2 | 0.2 | 0.2 | 0.2 |
| 15 | KSP-100 (Note 5) | 1.5 | 1.5 | 1.5 | 1.5 |
| 16 | Dipropylene glycol | 5.0 | 5.0 | 5.0 | 5.0 |
| 17 | Purified water | 43.0 | 43.0 | 43.0 | 43.0 |
| Total | | 100.0 | 100.0 | 100.0 | 100.0 |

| | | | | | |
|---|---|---|---|---|---|
| (Note 1) Shin-Etsu Chemical Co., Ltd.: Diphenylsiloxyphenyl trimethicone (Note 2) Shin-Etsu Chemical Co., Ltd.: Mixture of 70-80% dimethicone + 20-30% (dimethicone/polyglycerin-3) crosspolymer (Note 3) Shin-Etsu Chemical Co., Ltd.: Lauryl PEG-9 polydimethylsiloxyethyl dimethicone (Note 4) Shin-Etsu Chemical Co., Ltd.: KF-9909-treated coloring inorganic pigments, W: white, R: red, Y: yellow, B: black (Note 5) Shin-Etsu Chemical Co., Ltd.: (Vinyl dimethicone/methicone silsesquioxane) crosspolymer | | | | | |

### [Production method]

A: Ingredients 11 to 14 were mixed with a portion of ingredient 5 and the mixture was dispersed with a roll mill.
B: Ingredients 1 to 4, the remainder of ingredient 5, and ingredients 6 to 10 and 15 were dissolved by heating and were mixed together.
C: Ingredients 16 and 17 were mixed together and the mixture was heated.
D: The mixture obtained in C was gently added to the mixture obtained in B, and the resultant mixture was stirred to give an emulsion.
E: The mixture obtained in A was added to the emulsion obtained in D. The mixture was stirred, poured into a mold, and cooled to solidify. A stick-type W/O foundation was thus obtained.

The stick-type W/O foundations obtained were used by a panel of ten female experts to test (1) spreadability, (2) smoothness of film, (3) absence of glare, and (4) lightness of cosmetic film according to the ratings below. The scores were averaged and the results were evaluated based on the criteria below. The results are described in Table 6.

Furthermore, the cosmetic products were observed (5) after being allowed to stand at 40°C for one month and (6) after being allowed to stand at 5°C for one month. The results are described in Table 6.

### (Ratings)

| | |
|---|---|
| 5 Points: | Very good |
| 4 Points: | Good |
| 3 Points: | Fair |
| 2 Points: | Rather poor |
| 1 Point: | Poor |

The average score was evaluated based on the following ○ × criteria.

### (Evaluation criteria)

⊚: The average score is 4.5 points or more.
○: The average score is 3.5 points or more and less than 4.5 points.
△: The average score is 2.5 points or more and less than 3.5 points.
×: The average score is 1.5 points or more and less than 2.5 points.
××: The average score is less than 1.5 points.

**[Table 6]**

| No. | Evaluation items | Example 3 | Example 4 | Comparative Example 4 | Comparative Example 5 |
|---|---|---|---|---|---|
| (1) | Spreadability | ⊚ | ⊚ | ○ | ⊚ |
| (2) | Smoothness of film | ⊚ | ⊚ | △ | ⊚ |
| (3) | Absence of glare | ⊚ | ⊚ | × | ⊚ |
| (4) | Lightness of film | ○ | ⊚ | × | ○ |
| (5) | State of cosmetic product (40°C) | Stable | Stable | Stable | Separated oil |
| (6) | State of cosmetic product (5°C) | Stable | Stable | Stable | Separated oil |

As clear from Table 6, the foundations of Examples 3 and 4 as compared to Comparative Example 4 were demonstrated to have good spreadability, to be smooth and glare-free, and to form a light film after application. Furthermore, these foundations were shown to have excellent storage stability. The foundation of Comparative Example 5 had a good feel but was poor in storage stability.

### [Example 5] Sunscreen lotion (shaking)

| Composition | | % |
|---|---|---|
| 1. | Dimethylpolysiloxane (1.5 cs) | 22.5 |
| 2. | Dimethylpolysiloxane (6 cs) | 2 |
| 3. | Siloxane of Production Example 1 | 7 |
| 4. | KSG-18A (Note 1) | 3 |
| 5. | KF-6038 (Note 2) | 2 |
| 6. | Ethylhexyl methoxycinnamate | 7.5 |
| 7. | Diethylamino hydroxybenzoyl hexyl benzoate | 1 |
| 8. | Octocrylene | 2.5 |
| 9. | Disteardimonium hectorite | 1 |
| 10. | KP-545 (Note 3) | 2 |
| 11. | KSP-105 (Note 4) | 0.5 |
| 12. | SPD-T5 (Note 5) | 5 |
| 13. | SPD-Z5 (Note 6) | 10 |
| 14. | BG | 3 |
| 15. | Sodium citrate | 0.2 |
| 16. | Sodium chloride | 0.5 |
| 17. | Ethanol | 5 |
| 18. | Water | 25.3 |
| Total | | 100.0 |

| | | |
|---|---|---|
| (Note 1) Shin-Etsu Chemical Co., Ltd.: Mixture of 80-90% diphenylsiloxyphenyl trimethicone + 10-20% (dimethicone/phenyl vinyl dimethicone) crosspolymer (Note 2) Shin-Etsu Chemical Co., Ltd.: Lauryl PEG-9 polydimethylsiloxyethyl dimethicone (Note 3) Shin-Etsu Chemical Co., Ltd.: Solution of 70% cyclopentasiloxane + 30% (acrylates/dimethicone) copolymer (Note 4) Shin-Etsu Chemical Co., Ltd.: (Vinyl dimethicone/methicone silsesquioxane) crosspolymer (Note 5) Shin-Etsu Chemical Co., Ltd.: Dispersion of 40% titanium oxide fine particles in cyclopentasiloxane (Note 6) Shin-Etsu Chemical Co., Ltd.: Dispersion of 60% zinc oxide fine particles in cyclopentasiloxane | | |

### (Production method)

A: Ingredients 1 to 10 were mixed uniformly.
B: Ingredient 11 was added to the mixture obtained in A, and the resultant mixture was dispersed uniformly.
C: Ingredients 14 to 17 were added to ingredient 18 and were dissolved.
D: The mixture obtained in C was gradually added to the mixture obtained in B to form an emulsion, to which ingredients 12 and 13 were added to give a sunscreen lotion.

The sunscreen lotion obtained as described above spread lightly, left the skin dry without stickiness, and was free from changes due to temperature or aging, thus being shown to have excellent usability and stability.

### [Example 6] Sunscreen cream

| Composition | | % |
|---|---|---|
| 1. | Dimethylpolysiloxane (2 cs) | 22 |
| 2. | Siloxane of Production Example 2 | 8 |
| 3. | Squalane | 3 |
| 4. | KF-6105 (Note 1) | 4 |
| 5. | Ethylhexyl methoxycinnamate | 7.5 |
| 6. | t-Butyl methoxydibenzoylmethane | 3 |
| 7. | Polysilicone-15 | 1 |
| 8. | Distearyldimonium chloride | 1 |
| 9. | Tocophenol acetate | 0.1 |
| 10. | Ethanol | 1 |
| 11. | Sodium citrate | 0.5 |
| 12. | Magnesium sulfate | 0.5 |
| 13. | Preservative | 0.3 |
| 14. | Water | 48.1 |
| Total | | 100.0 |

| | | |
|---|---|---|
| (Note 1) Shin-Etsu Chemical Co., Ltd.: Lauryl polyglyceryl-3 polydimethylsiloxyethyl dimethicone | | |

### (Production method)

A: Ingredients 1 to 9 were mixed uniformly.
B: Ingredients 10 to 13 were dissolved uniformly into ingredient 14.
C: While performing stirring, the mixture obtained in B was gradually added to the mixture obtained in A and emulsified to give a sunscreen cream.

The sunscreen cream obtained as described above had a fine texture, spread lightly, gave a moist and dewy feel, and was free from stickiness and caught no sand, thus being shown to have very good usability. Furthermore, the sunscreen cream was free from changes due to temperature or aging, thus being shown to have excellent stability.

### [Example 7] Sunscreen cream

| Composition | | % |
|---|---|---|
| 1. | Decamethylcyclopentasiloxane | 8 |
| 2. | Siloxane of Production Example 1 | 8 |
| 3. | KSG-310 (Note 1) | 3.5 |
| 4. | KSG-18A (Note 2) | 3 |
| 5. | KF-6048 (Note 3) | 1.5 |
| 6. | KF-7312J (Note 4) | 3 |
| 7. | Ethylhexyl methoxycinnamate | 7.5 |
| 8. | t-Butyl methoxydibenzoylmethane | 3 |
| 9. | Ethylhexyl salicylate | 3 |
| 10. | BG | 5 |
| 11. | Sodium citrate | 0.5 |
| 12. | Sodium chloride | 1 |
| 13. | Preservative | 0.3 |
| 14. | Water | 52.7 |
| Total | | 100.0 |

| | | |
|---|---|---|
| (Note 1) Shin-Etsu Chemical Co., Ltd.: Mixture of 65-75% mineral oil + 25-35% (PEG-15/lauryl dimethicone) crosspolymer (Note 2) Shin-Etsu Chemical Co., Ltd.: Mixture of 80-90% diphenylsiloxyphenyl trimethicone + 10-20% (dimethicone/phenyl vinyl dimethicone) crosspolymer (Note 3) Shin-Etsu Chemical Co., Ltd.: Cetyl PEG/PPG-10/1 dimethicone (Note 4) Shin-Etsu Chemical Co., Ltd.: Solution of 50% trimethylsiloxysilicate in cyclopentasiloxane | | |

### (Production method)

A: Ingredients 1 to 9 were mixed uniformly.
B: Ingredients 10 to 13 were dissolved uniformly into ingredient 14.
C: While performing stirring, the mixture obtained in B was gradually added to the mixture obtained in A and emulsified to give a sunscreen cream.

The sunscreen cream obtained as described above spread lightly, gave a dewy feel without stickiness, and formed a uniform and highly water-repellent film, thus offering long-lasting cosmetic and ultraviolet protective effects. Furthermore, the sunscreen cream was free from changes due to temperature or aging. Thus, the sunscreen cream was shown to have excellent usability and stability.

### [Example 8] Non-aqueous mousse foundation

| Composition | | % |
|---|---|---|
| 1. | KSG-240 (Note 1) | 18 |
| 2. | Dimethylpolysiloxane (6 CS) | 7 |
| 3. | Siloxane of Production Example 2 | 5 |
| 4. | Ethylhexyl methoxycinnamate | 5 |
| 5. | Jojoba oil | 1 |
| 6. | Silylated silicic anhydride (Note 2) | 0.75 |
| 7. | KTP-09R (Note 3) | 0.2 |
| 8. | KTP-09Y (Note 3) | 1 |
| 9. | KTP-09B (Note 3) | 0.02 |
| 10. | KTP-09W (Note 3) | 5 |
| 11. | KF-9909 (Note 4) treated talc | 11.55 |
| 12. | KF-7312J (Note 5) | 4 |
| 13. | Decamethylcyclopentasiloxane | 25.28 |
| 14. | KSP-411 (Note 6) | 6 |
| 15. | KMP-590 (Note 7) | 3 |
| 16. | Spherical poly(alkyl methacrylate) | 7 |
| 17. | Antioxidant | 0.2 |
| Total | | 100.0 |

| | | |
|---|---|---|
| (Note 1) Shin-Etsu Chemical Co., Ltd.: Mixture of 75-85% cyclopentasiloxane + 15-25% (dimethicone/(PEG-10/15)) crosspolymer (Note 2) NIPPON AEROSIL CO., LTD.: Aerosil R-972, surface-hydrophobized fumed silica (Note 3) Shin-Etsu Chemical Co., Ltd.: KF-9909-treated coloring inorganic pigments, W: white, R: red, Y: yellow, B: black (Note 4) Shin-Etsu Chemical Co., Ltd.: Triethoxysilylethyl polydimethylsiloxyethylhexyl dimethicone (Note 5) Shin-Etsu Chemical Co., Ltd.: Solution of 50% trimethylsiloxysilicate in cyclopentasiloxane (Note 6) Shin-Etsu Chemical Co., Ltd.: Polysilicone-1 crosspolymer (Note 7) Shin-Etsu Chemical Co., Ltd.: Polymethylsilsesquioxane | | |

### (Production method)

Ingredients 1 to 10 were mixed uniformly by roller treatment. Ingredients 11 to 17 were added to the mixture, and the resultant mixture was mixed uniformly to give a non-aqueous mousse foundation.

The non-aqueous mousse foundation obtained as described above had a mousse-like appearance, was firm, spread lightly, had an excellent feeling on use without stickiness or oily feel, and offered very long-lasting cosmetic effects. Furthermore, the foundation was free from oil bleeding or the like due to temperature or aging, and had excellent stability.

### [Example 9]

### Non-aqueous eye wrinkle cream

| Composition | | % |
|---|---|---|
| 1. | KSG-210 (Note 1) | 5 |
| 2. | KSG-41A (Note 2) | 7 |
| 3. | KSG-15 (Note 3) | 55 |
| 4. | KF-4422 (Note 4) | 8 |
| 5. | Siloxane of Production Example 2 | 6 |
| 6. | Jojoba oil | 2 |
| 7. | KSP-101 (Note 5) | 12 |
| 8. | KF-9028 (Note 6) | 5 |
| Total | | 100.0 |

| | | |
|---|---|---|
| (Note 1) Shin-Etsu Chemical Co., Ltd.: Mixture of 70-80% dimethicone + 20-30% (dimethicone/(PEG-10/15)) crosspolymer (Note 2) Shin-Etsu Chemical Co., Ltd.: Mixture of 70-80% mineral oil + 20-30% (vinyl dimethicone/lauryl dimethicone) crosspolymer (Note 3) Shin-Etsu Chemical Co., Ltd.: Mixture of 90-96% cyclopentasiloxane + 4-10% (dimethicone/vinyl dimethicone) crosspolymer (Note 4) Shin-Etsu Chemical Co., Ltd.: Ethyl methicone (Note 5) Shin-Etsu Chemical Co., Ltd.: (Vinyl dimethicone/methicone silsesquioxane) crosspolymer (Note 6) Shin-Etsu Chemical Co., Ltd.: Solution of 20% dimethicone (highly polymerized) in cyclopentasiloxane | | |

### (Production method)

A: Ingredients 1 to 8 were mixed uniformly to give a non-aqueous eye wrinkle cream.

The non-aqueous eye wrinkle cream obtained as described above spread lightly, had no stickiness or oily feel, and offered a moist and comfortable feel to the skin. Furthermore, the eye wrinkle cream was free from changes due to temperature or aging, thus being shown to have excellent stability.

### [Example 10] Wash-off pack cosmetic product

| Composition | | % |
|---|---|---|
| 1. | Dimethylpolysiloxane (6 CS) | 3 |
| 2. | Siloxane of Production Example 2 | 3 |
| 3. | KF-6100 (Note 1) | 2 |
| 4. | Kaolin | 30 |
| 5. | Carbomer | 0.4 |
| 6. | BG | 10 |
| 7. | Glycerin | 20 |
| 8. | Preservative | 0.1 |
| 9. | Fragrance | 0.1 |
| 10. | Water | 31.4 |
| Total | | 100.0 |

| | | |
|---|---|---|
| (Note 1) Shin-Etsu Chemical Co., Ltd.: Polyglyceryl-3 disiloxane dimethicone | | |

### (Production method)

A: Ingredients 1 to 3 and 8 were mixed.
B: Ingredients 5 to 7 and 10 were mixed uniformly, and subsequently ingredients 4 and 9 were mixed. The resultant mixture was stirred.
C: The mixture obtained in A was added to the mixture obtained in B to form an emulsion. A paste-like wash-off pack cosmetic product was thus obtained.

The wash-off pack cosmetic product obtained as described above spread lightly during application and offered excellent cleansing effects. After the pack cosmetic product was rinsed off, the skin had a moist and smooth feel without stickiness. The pack cosmetic product thud had a very good feeling on use and was also shown to have excellent stability.

### [Example 11] Eye color product

| Composition | | % |
|---|---|---|
| 1. | TMF-1.5 (Note 1) | 25.5 |
| 2. | KP-550 (Note 2) | 20 |
| 3. | KP-561P (Note 3) | 2 |
| 4. | KSP-441 (Note 4) | 6 |
| 5. | Isododecane | 3 |
| 6. | Siloxane of Production Example 1 | 5 |
| 7. | Vaseline | 5 |
| 8. | KSG-320 (Note 5) | 5 |
| 9. | Amorphous silicic anhydride (Note 6) | 1 |
| 10. | Barium sulfate | 5 |
| 11. | Organic pigment | 0.2 |
| 12. | KTP-09Y (Note 7) | 1 |
| 13. | KTP-09W (Note 7) | 1 |
| 14. | KF-9909 (Note 8) treated mica titanium | 20 |
| 15. | Tocopherol | 0.2 |
| 16. | Fragrance | 0.1 |
| Total | | 100.0 |

| | | |
|---|---|---|
| (Note 1) Shin-Etsu Chemical Co., Ltd.: Methyl trimethicone (Note 2) Shin-Etsu Chemical Co., Ltd.: Solution of 60% isododecane + 40% (acrylates/dimethicone) copolymer (Note 3) Shin-Etsu Chemical Co., Ltd.: (Acrylates/stearyl acrylate/dimethicone methacrylate) copolymer (Note 4) Shin-Etsu Chemical Co., Ltd.: Polysilicone-22 (Note 5) Shin-Etsu Chemical Co., Ltd.: Mixture of 70-80% isododecane + 20-30% (PEG-15/lauryl dimethicone) crosspolymer (Note 6) NIPPON AEROSIL CO., LTD.: Aerosil R-972, surface-hydrophobized fumed silica (Note 7) Shin-Etsu Chemical Co., Ltd.: KF-9909-treated coloring inorganic pigments, W: white, Y: yellow (Note 8) Shin-Etsu Chemical Co., Ltd.: Triethoxysilylethyl polydimethylsiloxyethylhexyl dimethicone | | |

### (Production method)

A: Ingredients 1 to 9 were mixed together to give a uniform dispersion.
B: Ingredients 10 to 16 were added to the mixture obtained in A. The resultant mixture was uniformly dispersed to give an eye color product.

The eye color product obtained as described above was easy to remove, spread lightly, and did not feel oily or powdery when used. Furthermore, the eye color product had good water resistance, water repellency, and perspiration resistance and thus had good durability and was resistant to coming off. Furthermore, the eye color product was free from changes due to temperature or aging, thus being shown to have excellent stability.

### [Example 12] Eyeliner

| Composition | | % |
|---|---|---|
| 1. | Siloxane of Production Example 2 | 10 |
| 2. | Decamethylcyclopentasiloxane | 12 |
| 3. | KF-96A-6CS (Note 1) | 5 |
| 4. | KSG-42A (Note 2) | 5 |
| 5. | Jojoba oil | 2 |
| 6. | KF-6038 (Note 3) | 3 |
| 7. | KF-9901 (Note 4) treated black iron oxide | 20 |
| 8. | Ethanol | 5 |
| 9. | Preservative | 0.1 |
| 10. | Water | 37.9 |
| Total | | 100.0 |

| | | |
|---|---|---|
| (Note 1) Shin-Etsu Chemical Co., Ltd.: Dimethicone (6 cs) (Note 2) Shin-Etsu Chemical Co., Ltd.: Mixture of 75-85% isododecane + 15-25% (vinyl dimethicone/lauryl dimethicone) crosspolymer (Note 3) Shin-Etsu Chemical Co., Ltd.: Lauryl PEG-9 polydimethylsiloxyethyl dimethicone (Note 4) Shin-Etsu Chemical Co., Ltd.: Hydrogen dimethicone | | |

### (Production method)

A: Ingredients 1 to 6 were mixed under heating conditions, and ingredient 7 was added and dispersed uniformly.
B: Ingredients 8 to 10 were dissolved under heating conditions.
C: While performing stirring, the mixture obtained in B was gradually added to the mixture obtained in A to form an emulsion. An eyeliner was thus obtained.

The eyeliner obtained as described above spread lightly without feeling oily or powdery, and gave a dewy and refreshing feeling on use. Furthermore, the eyeliner had good water resistance, water repellency, and perspiration resistance and thus had good durability and was resistant to coming off. Furthermore, the eyeliner was free from changes due to temperature or aging, thus being shown to have excellent stability.

### [Example 13] W/O cleansing cream

| Composition | | % |
|---|---|---|
| 1. | Dimethylpolysiloxane (6 CS) | 10 |
| 2. | KF-54 (Note 1) | 15 |
| 3. | Mineral oil | 8 |
| 4. | Isostearic acid | 1 |
| 5. | Siloxane of Production Example 1 | 10 |
| 6. | Siloxane of Production Example 2 | 1 |
| 7. | Dextrin fatty acid ester | 0.8 |
| 8. | KF-6017 (Note 2) | 4 |
| 9. | Glycerin | 10 |
| 10. | Sodium citrate | 0.2 |
| 11. | Sodium chloride | 1 |
| 12. | Preservative | 0.1 |
| 13. | Fragrance | 0.1 |
| 14. | Purified water | 38.8 |
| Total | | 100.0 |

| | | |
|---|---|---|
| (Note 1) Shin-Etsu Chemical Co., Ltd.: Diphenyl dimethicone (Note 2) Shin-Etsu Chemical Co., Ltd.: PEG-10 dimethicone | | |

A: Ingredients 1 to 8 were mixed under heating conditions.
B: Ingredients 9 to 12 and 14 were dissolved under heating conditions.
C: While performing stirring, the mixture obtained in B was gradually added to the mixture obtained in A to form an emulsion, which was then cooled. Ingredient 13 was added. A W/O cleansing cream was thus obtained.

The W/O cleansing cream obtained as described above had a fine texture, spread lightly without feeling sticky or oily, gave a moist, dewy, and refreshing feeling on use, and offered high cleansing effects. Furthermore, the W/O cleansing cream was free from changes due to temperature or aging, thus being shown to have excellent stability.

### [Example 14] Lip cream

| Composition | | % |
|---|---|---|
| 1. | Dextrin (palmitate/ethylhexanoate) (Note 1) | 9 |
| 2. | Siloxane of Production Example 2 | 7 |
| 3. | KP-545 (Note 2) | 5 |
| 4. | KSG-43 (Note 3) | 8 |
| 5. | KF-6105 (Note 4) | 2 |
| 6. | Decamethylcyclopentasiloxane | 46 |
| 7. | 1,3-Butylene glycol | 5 |
| 8. | Purified water | 10.8 |
| 9. | Colorant | 1.2 |
| 10. | Titanium oxide-coated mica | 6 |
| Total | | 100.0 |

| | | |
|---|---|---|
| (Note 1) Chiba Flour Milling Co., Ltd.: Rheopearl TT (Note 2) Shin-Etsu Chemical Co., Ltd.: Solution of 70% cyclopentasiloxane + 30% (acrylates/dimethicone) copolymer (Note 3) Shin-Etsu Chemical Co., Ltd.: Mixture of 65-75% triethylhexanoin + 25-35% (vinyl dimethicone/lauryl dimethicone) crosspolymer (Note 4) Shin-Etsu Chemical Co., Ltd.: Lauryl polyglyceryl-3 polydimethylsiloxyethyl dimethicone | | |

### (Production method)

A: Ingredient 9 was mixed with a portion of ingredient 2 and the mixture was dispersed with a roller mill. The resultant dispersion was mixed with ingredient 1, the remainder of ingredient 2, and ingredients 3 to 6 under heating conditions.
B: Ingredients 7 and 8 were heated and added to the mixture obtained in A to form an emulsion, which was then cooled.
C: Ingredient 10 was added to the emulsion obtained in B. A lip cream was thus obtained.

The lip cream obtained spread lightly, was free from stickiness or oily feel, and formed a long-lasting film on the lips.

### [Example 15] Mascara

| Composition | | % |
|---|---|---|
| 1. | KF-6028 (Note 1) | 1 |
| 2. | Disteardimonium hectorite | 4 |
| 3. | Isododecane | 39.5 |
| 4. | KP-550 (Note 2) | 20 |
| 5. | Dextrin palmitate/ethylhexanoate (Note 3) | 3 |
| 6. | Ceresin | 2.5 |
| 7. | KP-562P (Note 4) | 2 |
| 8. | Beeswax | 2.5 |
| 9. | Siloxane of Production Example 1 | 5 |
| 10. | Hydrogenated lecithin | 0.5 |
| 11. | Silica | 3 |
| 12. | Talc | 12 |
| 13. | KTP-09B (Note 5) | 5 |
| Total | | 100.0 |

| | | |
|---|---|---|
| (Note 1) Shin-Etsu Chemical Co., Ltd.: Lauryl PEG-9 polydimethylsiloxyethyl dimethicone (Note 2) Shin-Etsu Chemical Co., Ltd.: Solution of 60% isododecane + 40% (acrylates/dimethicone) copolymer (Note 3) Chiba Flour Milling Co., Ltd.: Rheopearl TT (Note 4) Shin-Etsu Chemical Co., Ltd.: (Acrylates/behenyl acrylate/dimethicone methacrylate) copolymer (Note 5) Shin-Etsu Chemical Co., Ltd.: KF-9909-treated coloring inorganic pigment, B: black | | |

### (Production method)

A: Ingredients 1 to 3 were mixed uniformly.
B: Ingredients 4 to 10 were dissolved while performing heating and stirring, and the mixture obtained in A and crushed ingredients 11, 12, and 13 were added. The mixture was mixed uniformly and was then cooled.

The mascara obtained as described above was non-sticky, spread lightly, was easy to apply to the eyelashes, and offered very long-lasting cosmetic effects.

### [Example 16] Oil cleansing product

| Composition | | % |
|---|---|---|
| 1. | Mineral oil | 30 |
| 2. | Isopropyl myristate | 2 |
| 3. | Siloxane of Production Example 1 | 54.9 |
| 4. | PEG-6 diisostearate | 1 |
| 5. | Tocopherol acetate | 0.1 |
| 6. | PEG-20 glyceryl triisostearate | 10 |
| 7. | Glycerin | 1 |
| 8. | Water | 1 |
| Total | | 100.0 |

### (Production method)

A: Ingredients 1 to 6 were mixed uniformly.
B: Ingredients 7 and 8 were dissolved by stirring. The solution was added to the mixture obtained in A, and the resultant mixture was mixed uniformly to give an oil cleansing product.

The oil cleansing product obtained as described above was non-sticky, spread easily to form a uniform oil film, and had very high cleansing effects.

### [Example 17] Deodorant stick

| Composition | | % |
|---|---|---|
| 1. | Chlorohydroxyaluminum | 23 |
| 2. | Dimethylpolysiloxane (2 cs) | 33.5 |
| 3. | Siloxane of Production Example 2 | 3 |
| 4. | Stearyl alcohol | 8 |
| 5. | Talc | 14.88 |
| 6. | Fragrance | 0.1 |
| 7. | BHT | 0.02 |
| 8. | Paraffin (solid) | 2 |
| 9. | Mineral oil | 14.5 |
| 10. | KF-6048 (Note 1) | 1 |
| Total | | 1 100.0 |

| | | |
|---|---|---|
| (Note 1) Shin-Etsu Chemical Co., Ltd.: Cetyl PEG/PPG-10/1 dimethicone | | |

### (Production method)

A: Ingredients 2 to 4 and 7 to 10 were dissolved under heating conditions and mixed together.
B: Ingredients 1 and 5 were mixed uniformly with the mixture obtained in A. The resultant mixture was dispersed and mixed uniformly using a mixer.
C: Ingredient 6 was added, and the resultant mixture was mixed uniformly.
D: The mixture was poured into a mold and was cooled to solidify.

The deodorant stick obtained as described above was free from an excessively dry or sticky feeling and was excellent in long-lasting deodorizing effects and in stability.

### [Example 18] Non-aqueous deodorant cream

| Composition | | % |
|---|---|---|
| 1. | Decamethylcyclopentasiloxane | 25 |
| 2. | Siloxane of Production Example 1 | 5 |
| 3. | KSG-15 (Note 1) | 21.5 |
| 4. | Neopentyl glycol dioctanoate | 9.68 |
| 5. | BHT | 0.02 |
| 6. | Benzyl alcohol | 0.1 |
| 7. | Polyethylene | 3 |
| 8. | Ceresin | 6 |
| 9. | Dimethylsilylated silica | 0.5 |
| 10. | Trichlorohydrex glycine (Al/zirconium) | 19 |
| 11. | KSP-100 (Note 2) | 10 |
| 12. | Citric acid | 0.1 |
| 13. | Fragrance | 0.1 |
| Total | | 100.0 |

| | | |
|---|---|---|
| (Note 1) Shin-Etsu Chemical Co., Ltd.: Mixture of 90-96% cyclopentasiloxane + 4-10% (dimethicone/vinyl dimethicone) crosspolymer (Note 2) Shin-Etsu Chemical Co., Ltd.: (Vinyl dimethicone/methicone silsesquioxane) crosspolymer | | |

### (Production method)

A: Ingredients 1 to 8 were heated and mixed together uniformly.
B: Ingredient 9 was mixed uniformly with the mixture obtained in A. The resultant mixture was dispersed and mixed uniformly using a mixer.
C: Ingredients 10 to 12 were added to the mixture obtained in B, and the resultant mixture was mixed uniformly.
D: Ingredient 13 was added to the mixture obtained in C. The resultant mixture was mixed uniformly and was then loaded into a container.

The non-aqueous deodorant cream obtained as described above spread very smoothly and well and was free from an excessively dry or sticky feeling. Furthermore, the non-aqueous deodorant cream was excellent in long-lasting deodorizing effects.

### [Example 19] Hair oil

| Composition | | % |
|---|---|---|
| 1. | Siloxane of Production Example 2 | 68 |
| 2. | Hydrogenated polyisobutene | 10.3 |
| 3. | KF-7312J (Note 1) | 3 |
| 4. | Dimethiconol | 7 |
| 5. | Diethylhexyl succinate | 10 |
| 6. | KF-9030 (Note 2) | 1.5 |
| 7. | Tocopherol | 0.1 |
| 8. | Fragrance | 0.1 |
| Total | | 100.0 |

| | | |
|---|---|---|
| (Note 1) Shin-Etsu Chemical Co., Ltd.: Solution of 50% trimethylsiloxysilicate in cyclopentasiloxane (Note 2) Shin-Etsu Chemical Co., Ltd.: Dimethicone (gum solution) | | |

### (Production method)

A: Ingredients 1 to 8 were mixed uniformly to give a hair oil.

The hair oil obtained as described above spread lightly and gave gloss and smoothness to the hair.

### [Example 20] Lipstick

| Composition | | % |
|---|---|---|
| 1. | Dimethylpolysiloxane (2 cs) | 28.8 |
| 2. | Siloxane of Production Example 2 | 10 |
| 3. | Diisostearyl malate | 5 |
| 4. | Candelilla wax | 10 |
| 5. | Polyethylene | 7 |
| 6. | Microcrystalline wax | 2 |
| 7. | KF-7312J (Note 1) | 20 |
| 8. | KSG-43 (Note 2) | 5 |
| 9. | KF-6105 (Note 3) | 3 |
| 10. | Mica | 8 |
| 11. | Colorant | 1.2 |
| Total | | 100.0 |

| | | |
|---|---|---|
| (Note 1) Shin-Etsu Chemical Co., Ltd.: Solution of 50% trimethylsiloxysilicate in cyclopentasiloxane (Note 2) Shin-Etsu Chemical Co., Ltd.: Mixture of 65-75% triethylhexanoin + 25-35% alkyl branched/crosslinked dimethylpolysiloxane (Note 3) Shin-Etsu Chemical Co., Ltd.: Lauryl polyglyceryl-3 polydimethylsiloxyethyl dimethicone | | |

### (Production method)

A: Ingredient 11 was mixed with a portion of ingredient 2 and the mixture was dispersed with a roll mill. The resultant dispersion was mixed with ingredient 1, the remainder of ingredient 2, and ingredients 3 to 10 under heating conditions.
B: The mixture was poured into a mold and was cooled to solidify. A lipstick was thus obtained.

The lipstick obtained spread well, formed a uniform film, and had high water repellency to give long-lasting cosmetic effects. The lipstick was also shown to have excellent storage stability.

### [Example 21] Water-breaking W/O foundation

| Composition | | % |
|---|---|---|
| 1. | KSG-270 (Note 1) | 4 |
| 2. | KSG-18A (Note 2) | 1 |
| 3. | KF-6038 (Note 3) | 0.1 |
| 4. | Ethylhexyl methoxycinnamate | 7.5 |
| 5. | Dimethylpolysiloxane (2 cs) | 5.6 |
| 6. | Siloxane of Production Example 1 | 4.8 |
| 7. | KF-6115 (Note 4) | 0.3 |
| 8. | KTP-09W (Note 5) | 5.1 |
| 9. | KTP-09Y (Note 5) | 0.58 |
| 10. | KTP-09R (Note 5) | 0.25 |
| 11. | KTP-09B (Note 5) | 0.07 |
| 12. | Hydrophobized zinc oxide fine particles (Note 6) | 6 |
| 13. | BG | 8 |
| 14. | Sodium citrate | 0.2 |
| 15. | Magnesium sulfate | 0.5 |
| 16. | Water | 56 |
| Total | | 100.0 |

| | | |
|---|---|---|
| (Note 1) Shin-Etsu Chemical Co., Ltd.: Mixture of 80% diphenylsiloxyphenyl trimethicone + 20% dimethicone/(PEG-10/15)) crosspolymer (Note 2) Shin-Etsu Chemical Co., Ltd.: Mixture of 85% diphenylsiloxyphenyl trimethicone + 15% (dimethicone/phenyl vinyl dimethicone) crosspolymer (Note 3) Shin-Etsu Chemical Co., Ltd.: Lauryl PEG-9 polydimethylsiloxyethyl dimethicone (Note 4) Shin-Etsu Chemical Co., Ltd.: Lauryl polyglyceryl-3 polydimethylsiloxyethyl dimethicone (Note 5) Shin-Etsu Chemical Co., Ltd.: KF-9909-treated coloring inorganic pigments, W: white, R: red, Y: yellow, B: black (Note 6) Shin-Etsu Chemical Co., Ltd.: AES-3083-treated | | |

### (Production method)

| | |
|---|---|
| Preparation of oil phase: | Ingredients 1 to 6 were mixed uniformly. |
| Preparation of aqueous phase: | Ingredients 13 to 16 were mixed uniformly. |
| Preparation of paste phase: | Ingredients 8 to 12 were mixed in ingredients 6 and 7, and the mixture was dispersed using a three-roll mill. |

The aqueous phase was gently added to the oil phase to effect a phase change. After emulsification, the paste was mixed. A water-breaking W/O foundation was thus obtained.

The water breaking W/O foundation obtained was shown to have a good feeling on use, a good water-breaking feel, and good applicability.

### [Example 22] Hair treatment

| Composition | | % |
|---|---|---|
| 1. | KSG-18A (Note 1) | 1 |
| 2. | Cetanol | 2 |
| 3. | Cetyl octanoate | 3 |
| 4. | Butyl paraoxybenzoate | 0.1 |
| 5. | Siloxane of Production Example 2 | 1 |
| 6. | Behentrimonium chloride | 1 |
| 7. | Propylene glycol | 5 |
| 8. | Hydroxyethyl cellulose | 0.1 |
| 9. | Water | Balance |
| 10. | X-52-2328 (Note 2) | 4 |
| 11. | Fragrance | q.s. |
| Total | | 100.0 |

| | | |
|---|---|---|
| (Note 1) Shin-Etsu Chemical Co., Ltd.: Mixture of 80-90% diphenylsiloxyphenyl trimethicone + 10-20% (dimethicone/phenyl vinyl dimethicone) crosspolymer (Note 2) Shin-Etsu Chemical Co., Ltd.: Aminopropyl dimethicone | | |

### (Production method)

A: Ingredients 6 to 9 were heated to 70°C and were mixed uniformly.
B: Ingredients 1 to 5 were heated to 70°C and were mixed uniformly.
C: The mixture obtained in B was added to the mixture obtained in A. The resultant mixture was emulsified. After gradual cooling, ingredients 10 and 11 were added. A hair treatment was thus obtained.

The hair treatment obtained had a good feeling on use, was easy to apply, and had excellent storage stability.

The present invention is not limited to the embodiments described above, which are presented here for the purpose of illustration. Any embodiments having substantially the same constitution as the technical ideas set forth in the claims of the present invention and exhibiting similar working effects are encompassed within the technical scope of the present invention.

## Claims

1. A cosmetic product comprising an organopolysiloxane represented by formula (1) below: 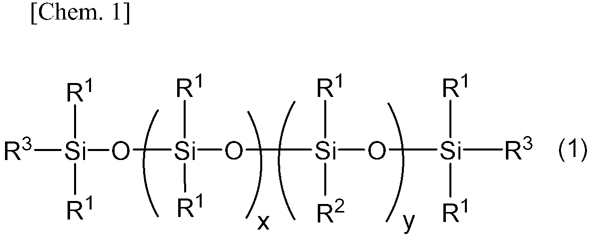 wherein R¹ independently at each occurrence is a C₁-C₁₆ alkyl group; R² is an organic group represented by formula (2) below: 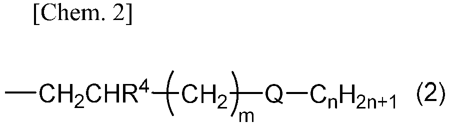 wherein R⁴ is a hydrogen atom or a methyl group,
Q is an organic group selected from linking groups represented by formulas (3) to (6) below: 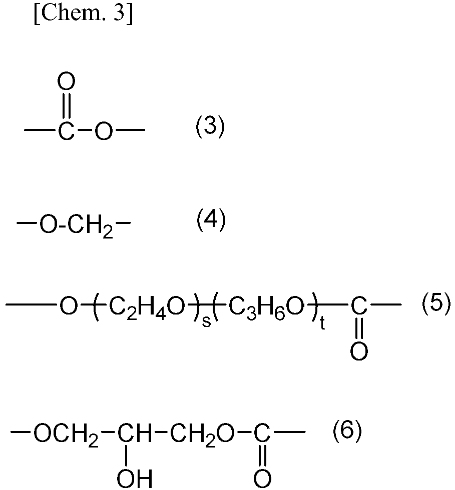 wherein s is an integer of 0 to 3, t is an integer of 0 to 3, and the respective oxyalkylene groups are optionally bonded blockwise or randomly,
when Q is formula (3), m is 8 and n is an integer of 1 to 22,
when Q is formula (4), m is an integer of 1 to 10 and n is an integer of 7 to 22,
when Q is formula (5), m is an integer of 1 to 10 and n is an integer of 7 to 22, and
when Q is formula (6), m is an integer of 1 to 10 and n is an integer of 7 to 22;
each R³ is R¹ or R²; x is an integer of 0 to 50; y is an integer of 0 to 50; at least one of R² or R³'s is an organic group represented by general formula (2); when y = 0, at least one of R³'s is R²; and the siloxane units with the subscripts x and y are optionally bonded blockwise or randomly.

2. The cosmetic product according to claim 1, wherein the organopolysiloxane has a surface tension of 28 mN/m or less and is nonvolatile.

3. The cosmetic product according to claim 1, further comprising an organic ultraviolet absorber.

4. The cosmetic product according to claim 3, wherein the organic ultraviolet absorber is one or more selected from ethylhexyl methoxycinnamate, diethylamino hydroxybenzoyl hexyl benzoate, octyl salicylate, polysilicone-15, t-butyl methoxydibenzoylmethane, oxybenzone, methylene bisbenzotriazolyl tetramethylbutylphenol, bisethylhexyloxyphenol methoxyphenyl triazine, and octocrylene.

5. The cosmetic product according to any one of claims 1 to 4, further comprising an oil ingredient that is solid at 25°C.

6. The cosmetic product according to claim 5, wherein the oil ingredient that is solid at 25°C is one or more selected from polyethylene, ceresin, ozokerite, candelilla wax, carnauba wax, beeswax, microcrystalline wax, stearyl alcohol, behenyl alcohol, and cetanol.
